# EUROPEAN PATENT SPECIFICATION

(11) **EP 2 046 288 B1**
(45) Date of publication and mention of the grant of the patent: **03.07.2013**
(21) Application number: 07730141.4
(22) Date of filing: 13.06.2007
(51) Int. Cl.: A61K 9/06, A61K 47/36, A61K 33/00, A61K 49/00, A61K 33/06, A61K 33/24, A61K 47/48, A61K 51/12, A61L 27/52, A61L 27/38, A61L 27/20, A61L 24/08, A61L 24/00, A61K 9/00, A61K 51/00

(54) **HYDROGELS CONTAINING LOW MOLECULAR WEIGHT ALGINATES AND BIOSTRUCTURES MADE THEREFROM**
HYDROGELE MIT NIEDERMOLEKULAREN ALGINATEN UND DARAUS HERGESTELLTE BIOSTRUKTUREN
HYDROGELS CONTENANT DES ALGINATES DE FAIBLE MASSE MOLÉCULAIRE ET BIOSTRUCTURES FABRIQUÉES À PARTIR DE CEUX-CI

(30) Priority: 14.07.2006 US 830959 P
(43) Date of publication of application: 15.04.2009
(60) Divisional application: 11163550.4
(73) Proprietor: FMC Biopolymer AS, 3013 Drammen (NO)
(72) Inventor: MELVIK, Jan Egil, 0281 Oslo (NO); GASEROD, Olav, 3053 Steinberg (NO)
(74) Representative: Read, David Graham
(86) International application number: PCT/EP2007/055856
(87) International publication number: WO 2008/006658

(56) References cited:
- EP-A- 1 618 883
- WO-A-00/21572
- WO-A-98/12228
- WO-A-99/15211
- WO-A-99/59549
- WO-A1-01/40314
- US-A- 5 626 870
- US-A1- 2004 197 373
- US-A1- 2005 053 655
- US-B1- 6 790 840

### SEQUENCE LISTING

<110> FMC Biopolymer AS Melvik, Jan E. Gaserod, Olav
<120> HYDROGELS CONTAINING LOW MOLECULAR WEIGHT ALGINATES AND BIOSTRUCTURES MADE THEREFROM
<130> FMC60521WO
<150> US 60/830,959 <151> 2006-07-14
<160> 22
<170> PatentIn version 3.4
<210> 1
   <211> 5
   <212> PRT
   <213> Artificial Sequence
<220>
   <223> Cell Adhesion Peptide 1
<400> 1
   Tyr Ile Gly Ser Arg
   1               5
<210> 2
   <211> 5
   <212> PRT
   <213> Artificial Sequence
<220>
   <223> Cell Adhesion Peptide 2
<400> 2
   Ile Lys Val Ala Val
   1               5
<210> 3
   <211> 4
   <212> PRT
   <213> Artificial sequence
<220>
   <223> cell Adhesion Peptide 3
<400> 3
   Arg Glu Asp Val
   1
<210> 4
   <211> 4
   <212> PRT
   <213> Artificial sequence
<220>
   <223> Cell Adhesion Peptide 4
<400> 4
   Asp Gly Glu Ala
   1
<210> 5
   <211> 6
   <212> PRT
   <213> Artificial sequence
<220>
   <223> cell Adhesion Peptide 5
<400> 5
   Val Gly Val Ala Pro Gly
   1               5
<210> 6
   <211> 5
   <212> PRT
   <213> Artificial Sequence
<220>
   <223> Cell Adhesion Peptide 6
<400> 6
   Gly Arg Gly Asp Ser
   1 5
<210> 7
   <211> 4
   <212> PRT
   <213> Artificial Sequence
<220>
   <223> cell Adhesion Peptide 7
<400> 7
   Arg Gly Asp Val
   1
<210> 8
   <211> 5
   <212> PRT
   <213> Artificial Sequence
<220>
   <223> Cell Adhesion Peptide 8
<400> 8
   Pro Asp Ser Gly Arg
   1 5
<210> 9
   <211> 7
   <212> PRT
   <213> Artificial Sequence
<220>
   <223> cell Adhesion Peptide 9
<400> 9
   Arg Tyr Val Val Leu Pro Arg
   1 5
<210> 10
   <211> 6
   <212> PRT
   <213> Artificial sequence
<220>
   <223> Cell Adhesion Peptide 10
<400> 10
   Leu Gly Thr Ile Pro Gly
   1 5
<210> 11
   <211> 4
   <212> PRT
   <213> Artificial sequence
<220>
   <223> Cell Adhesion Peptide 11
<400> 11
   Arg Gly Asp Ser
   1
<210> 12
   <211> 4
   <212> PRT
   <213> Artificial Sequence
<220>
   <223> Cell Adhesion Peptide 12
<400> 12
   Arg Gly Asp Phe
   1
<210> 13
   <211> 12
   <212> PRT
   <213> Artificial Sequence
<220>
   <223> Cell Adhesion Peptide 13
<400> 13
   His His Leu Gly Gly Ala Leu Gln Ala Gly Asp Val
   1 5 10
<210> 14
   <211> 4
   <212> PRT
   <213> Artificial Sequence
<220>
   <223> Cell Adhesion Peptide 14
<400> 14
   Val Thr Cys Gly
   1
<210> 15
   <211> 4
   <212> PRT
   <213> Artificial Sequence
<220>
   <223> Cell Adhesion Peptide 15
<400> 15
   Ser Asp Gly Asp
   1
<210> 16
   <211> 6
   <212> PRT
   <213> Artificial sequence
<220>
   <223> Cell Adhesion Peptide 16
<400> 16
   Gly Arg Glu Asp Val Tyr
   1 5
<210> 17
   <211> 5
   <212> PRT
   <213> Artificial sequence
<220>
   <223> Cell Adhesion Peptide 17
<400> 17
   Gly Arg Gly Asp Tyr
   1 5
<210> 18
   <211> 6
   <212> PRT
   <213> Artificial Sequence
<220>
   <223> cell Adhesion Peptide 18
<400> 18
   Gly Arg Gly Asp Ser Pro
   1 5
<210> 19
   <211> 4
   <212> PRT
   <213> Artificial sequence
<220>
   <223> Cell adhesion Peptide 19
<400> 19
   Val Ala Pro Gly
   1
<210> 20
   <211> 9
   <212> PRT
   <213> Artificial Sequence
<220>
   <223> cell Adhesion Peptide 20
<400> 20
   Gly Gly Gly Gly Arg Gly Asp Ser Pro
   1 5
<210> 21
   <211> 8
   <212> PRT
   <213> Artificial sequence
<220>
   <223> Cell Adhesion Peptide 21
<400> 21
   Gly Gly Gly Gly Arg Gly Asp Tyr
   1 5
<210> 22
   <211> 6
   <212> PRT
   <213> Artificial Sequence
<220>
   <223> Cell Adhesion Peptide 22
<400> 22
   Phe Thr Leu Cys Phe Asp
   1 5

## Description

### FIELD OF THE INVENTION

The present invention is directed to a hydrogel comprising an alginate having a low molecular weight (e.g., less than 75,000 Daltons), wherein the alginate is present in a high concentration (e.g., greater than 2.5% by weight of the hydrogel). The present invention is also directed to methods of making and using the hydrogel, as well as products containing the hydrogel.

### BACKGROUND OF THE INVENTION

Alginates are hydrophilic marine biopolymers with the unique ability to form heat-stable gels that can develop and set at physiologically relevant temperatures. Alginates are a family of non-branched binary copolymers of 1-4 glycosidically linked β-D-mannuronic acid (M) and α-L-guluronic acid (G) residues. The relative amount of the two uronic acid monomers and their sequential arrangement along the polymer chain vary widely, depending on the origin of the alginate, seaweed species, plant age, and part of the seaweed (e.g., stem, leaf). Alginic acid is substantially insoluble in water. It forms water-soluble salts with alkali metals, such as sodium, potassium, and, lithium; magnesium; ammonium; and the substituted ammonium cations derived from lower amines, such as methyl amine, ethanol amine, diethanol amine, and triethanol amine. The salts are soluble in aqueous media above pH 4, but are converted to alginic acid when the pH is lowered below about pH 4. A thermo-irreversible water-insoluble alginate gel is formed in the presence of gel-forming ions, e.g. calcium, barium, strontium, zinc, copper(+2), aluminum, and mixtures thereof, at appropriate concentrations. Alginate gels can be hydrogels, i.e. cross-linked alginate polymers that contain large amounts of water without dissolution. Applications with the hydrogels of alginates include cell or tissue encapsulation, tissue engineering, drug delivery and others.

EP1618883, US6790840, WO 9915211, US5626870, WO01/40314, US 2004/197373, WO 99/59549, US2005/053655, WO00/21572, WO98/12228 disclose various alginate compositions.

There is an interest in developing new encapsulation technologies that produce a stronger, more stable and resistant hydrogel for use in various medical applications. A stronger gel network may avoid the necessity to coat alginate gel beads with poly-cations that are often used to increase the strength of the bead. There is also a need to develop encapsulation technologies that reduce swelling properties, to design alginate structures with controlled biodegradability, to produce new contrast agents and radio-opaque materials with an increased density of barium ions, and to develop bioresorbable therapeutic compositions. This invention addresses these needs and others.

### SUMMARY OF THE INVENTION

The present invention further provides stable hydrogels comprising an alginate having a molecular weight less than 50,000 Daltons, wherein the alginate is present in an amount of at least 4% by weight of the hydrogel according to claim 1.

The present invention further provides non-oxidized hydrogels comprising an alginate having a molecular weight less than 50,000 Daltons and at least one of cells, pharmaceutically active agents, nutritional active agents, tissue, drugs, food, cosmetic agents, or radioactive isotopes, wherein the alginate is present in an amount of at least 4% by weight of the hydrogel.

The present invention further provides hydrogels comprising an alginate having a molecular weight greater than 10,000 Daltons and less than 75,000 Daltons, wherein the alginate is present in an amount of at least 4% by weight of the hydrogel, wherein said hydrogel further comprises cells or tissue.

The present invention further provides hydrogels formed by a method comprising:
a) forming a dispersion by mixing i) a solution comprising a soluble alginate with an insoluble gel particles or ii) immediately soluble alginate, insoluble gel particles and a solvent, and
b) dispensing the dispersion whereby the dispersion forms a hydrogel; wherein said soluble alginate or immediately soluble alginate has a molecular weight less than 75,000 Daltons, wherein the soluble alginate is present in an amount of at least 2.5% by weight of the hydrogel;

The present invention further provides beads comprising the hydrogels of the invention according to claim 22.

The present invention further provides beads comprising an alginate having a molecular weight less than 75,000 Daltons, wherein said alginate is present in an amount of at least 4% by weight of the gel.

The present invention further provides methods of making a hydrogel or bead of the invention, comprising the steps of preparing a solution of the alginate and adding said alginate solution to a solution comprising gelling cations to form said hydrogel.

The present invention further provides methods of making a hydrogel of the invention, comprising mixing a compound containing gelling cations into a solution containing the alginate, where said cations are released at a desired rate to said alginate solution to form said hydrogel.

The present invention further provides the products of the methods of the invention according to claims 23-24.

The present invention further provides implantable medical devices comprising a hydrogel of the invention according to claim 25.

The present invention further provides methods of implanting a bead or implantable medical device of the invention in patient in need thereof, comprising implanting a implantable medical device in said patient according to claims 26, 27.

The present invention further provides methods of implanting cells in need thereof, comprising implanting a hydrogel, bead, or implantable medical device of the invention, wherein said hydrogel of said hydrogel, bead, or implantable medical device further comprises cells.

The present invention further provides drug delivery formulations comprising the hydrogels or beads of the invention.

The present invention further provides contrast agents or radio-opaque materials comprising an alginate having a molecular weight less than 75,000 Daltons and barium ionically bound to said alginate, wherein: said alginate is present in an amount of at least 2.5% by weight of the gel; and said barium in the hydrogel is used as a radio-opaque material according to claim 29.

The present invention further provides methods of blocking blood vessels by using a hydrogel or beads of the invention, wherein said hydrogel or said beads are bioresorbable.

The present invention further provides an embolic therapeutic composition comprising the hydrogels or beads of the invention, wherein said hydrogel or said beads are bioresorbable according to claim 31.

The present invention further provides methods of forming a hydrogel of the invention, comprising:
a) forming a dispersion by mixing i) a solution comprising a soluble alginate with an insoluble gel particles or ii) immediately soluble alginate, insoluble gel particles and a solvent, and
b) dispensing the dispersion whereby the dispersion forms a hydrogel; wherein said soluble alginate has a molecular weight less than 75,000 Daltons, wherein the soluble alginate is present in an amount of at least 2.5% by weight of the hydrogel according to claim 32.

### BRIEF DESCRIPTION OF THE FIGURES

**Figure 1** depicts the effect of low molecular weight (MW) alginate on the swelling of alginate beads with time in saline.
**Figure 2** depicts the mechanical strength of beads made of a solution of 2 % Protanal LF 10/60 alone or in combination with 0 to 5 % highly degraded alginate.
**Figure 3** depicts the mechanical strength of beads made of 2 % LF 10/60 alone or in combination of 0 to 5 % of low molecular weight alginate (degraded Protanal LFR 5/60).
**Figure 4** depict the mechanical resistance of alginate beads (bead diameters was approximately 3 mm) as a function of compression.

### DETAILED DESCRIPTION OF THE INVENTION

In the present invention we have discovered that using alginates with a lower molecular weight than what has been suggested earlier may give advantages as compared to what has been previously known. The use of this invention is in the field of cell (tissue) encapsulation, drug delivery and in tissue engineering applications. For example this discovery allows the manufacturing of alginate gels that are more condensed than that resulting from current gelling technologies. This means that the gel is more resistant, stronger and more stable. Also, the stronger gel network may avoid the necessity to coat alginate gel beads with poly-cations that is usually used to increase the strength of the bead. We have also found that very low molecular alginate qualities may be used to make biodegradable structures for tissue engineering and sustained drug delivery applications. This may be achieved as the increased structure strength may again be lost if the molecular weight is degraded beyond a certain level. We have also discovered that alginate degraded to a low MW may be used in combination with higher MW alginates to increase alginate structure stability, including reducing swelling properties, and to make alginate structures with controlled biodegradability. Biodegradability can be changed by manipulating the type of alginate used, its molecular weight and the amount to be used in the biostructure.

Another aspect of the current invention is also to make biostructures with an increased density of cross-linked divalent cations like Ca²⁺, Ba²⁺ or Sr²⁺. One particular use of this would be to manufacture increased radio-opaque alginate structures using Ba²⁺ as cross-linking ion or contrast agents.

In some embodiments, the molecular weight is weight-average molecular weight. Weight-average molecular weight can be determined by Size Exclusion Chromatography with Multiple Angle Laser Light Scatter Detection (SEC-MALS).

Accordingly, in a first aspect, the present invention provide a hydrogel comprising an alginate having a molecular weight less than 75,000 Daltons, wherein the alginate is present in an amount of at least 2.5% by weight of the gel.

In a second aspect, the present invention further provides a stable hydrogel comprising an alginate having a molecular weight less than 50,000 Daltons, wherein the alginate is present in an amount of at least 4% by weight of the hydrogel. As used herein, the term "stable hydrogel" means at least 90% of the hydrogel does not dissolve in physiological fluid in less than 90 days. In some embodiments, at least 80% of the hydrogel does not dissolve in physiological fluid in less than 90 days. In some embodiments, at least 80% of the hydrogel does not dissolve in physiological fluid in less than 60 days. In some embodiments, at least 80% of the hydrogel does not dissolve in physiological fluid in less than 30 days. In some embodiments, at least 80% of the hydrogel does not dissolve under physiological conditions in less than 30 days. In some embodiments, at least 80% of the hydrogel does not dissolve under physiological conditions in less than 60 days. In some embodiments, at least 80% of the hydrogel does not dissolve under physiological conditions in less than 90 days. As used herein, "physiological conditions" refers to physiological fluid at physiological temperature.

In a third aspect, the present invention further provides a non-oxidized hydrogel comprising an alginate having a molecular weight less than 50,000 Daltons and at least one of cells, pharmaceutically active agents, nutritional active agents, tissue, drugs, food, cosmetic agents, or radioactive isotopes, wherein the alginate is present in an amount of at least 4% by weight of the hydrogel. As used herein, the term "non-oxidized alginate" means that the alginate has a degree of oxidation of less than 20%. The degree of oxidation can be measured by methods known in the art, for example, by measuring the number of aldehyde groups using t-butyl carbazate (see e.g., Bouhadir, Polymer 1999, 40, 3575-84).

In a fourth aspect, the present invention further provides a hydrogel comprising an alginate having a molecular weight greater than 10,000 Daltons and less than 75,000 Daltons, wherein the alginate is present in an amount of at least 4% by weight of the hydrogel, wherein said hydrogel further comprises cells or tissue.

It is appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination. For example, the molecular weights and concentrations for the alginates that are described infra are intended to be provided for each aspect of the invention, where appropriate. It is further indeed that these embodiments can be combined in any suitable combination within each aspect of the invention.

In some embodiments, the alginate is present in an amount of at least 3.0%, at least about 4.0%, at least about 5.0%, at least about 6.0%, at least about 7.0%, at least about 8.0% or at least about 9.0% by weight of the gel.

In some embodiments, the alginate has a molecular weight less than 70,000 Daltons, less than 65,000 Daltons, less than 50,000 Daltons, less than 45,000 Daltons, less than 40,000 Daltons, less than 35,000 Daltons, less than 30,000 Daltons, less than 25,000 Daltons, less than 20,000 Daltons, less than 15,000 Daltons, less than 10,000 Daltons, less than 5,000 Daltons, less than 3,000 Daltons, less than 1,000 Daltons, or less than 350 Daltons.

In some embodiments, the alginate has a molecular weight than greater than 3,000 Daltons, greater than 5,000 Daltons, greater than 7,000 Daltons, greater than 10,000 Daltons, greater than 15,000 Daltons, or greater than 20,000 Daltons.

In some embodiments, the alginate has a molecular weight of less than about 75,000 Daltons, but greater than about 7,000 Daltons. In some embodiments, the alginate has a molecular weight of less than about 75,000 Daltons, but greater than about 10,000 Daltons. In some embodiments, the alginate has a molecular weight of less than about 75,000 Daltons, but greater than about 15,000 Daltons. In some embodiments, the alginate has a molecular weight of less than about 75,000 Daltons, but greater than about 20,000 Daltons.

In some embodiments, the alginate has a molecular weight below 50 kDa. In some embodiments, the alginate has a molecular weight below 6 kD.

In some embodiments, the alginate is present in an amount of at least 2.50% by weight of the gel; and the alginate has a molecular weight below 50 kDa. In some embodiments, the alginate is present in an amount of at least 3.0% by weight of the gel; and the alginate has a molecular weight below 50 kDa. In some embodiments, the alginate is present in an amount of at least 4.0% by weight of the gel; and the alginate has a molecular weight below 50 kDa. In some embodiments, the alginate is present in an amount of at least 5.0% by weight of the gel; and the alginate has a molecular weight below 50 kDa. In some embodiments, the alginate is present in an amount of at least 6.0% by weight of the gel; and the alginate has a molecular weight below 50 kDa.

In some embodiments, the alginate is present in an amount of at least 2.50% by weight of the gel; and the alginate has a molecular weight below 6 kDa. In some embodiments, the alginate is present in an amount of at least 3.0% by weight of the gel; and the alginate has a molecular weight below 6 kDa. In some embodiments, the alginate is present in an amount of at least 4.0% by weight of the gel; and the alginate has a molecular weight below 6 kDa. In some embodiments, the alginate is present in an amount of at least 5.0% by weight of the gel; and the alginate has a molecular weight below 6 kDa. In some embodiments, the alginate is present in an amount of at least 6.0% by weight of the gel; and the alginate has a molecular weight below 6 kDa.

In some embodiments, the alginate has a molecular weight of less than about 50,000 Daltons, but greater than about 3,000 Daltons. In some embodiments, the alginate has a molecular weight of less than about 50,000 Daltons, but greater than about 5,000 Daltons. In some embodiments, the alginate has a molecular weight of less than about 50,000 Daltons, but greater than about 7,000 Daltons. In some embodiments, the alginate has a molecular weight of less than about 50,000 Daltons, but greater than about 10,000 Daltons. In some embodiments, the alginate has a molecular weight of less than about 50,000 Daltons, but greater than about 15,000 Daltons. In some embodiments, the alginate has a molecular weight between 20,000 Daltons to 50,000 Daltons.

In some embodiments, the alginate has a molecular weight of less than about 6,000 Daltons, but greater than about 3,000 Daltons.

In some embodiments, the alginate comprises at least one of: (i) an alginate consisting of G-blocks, (ii) an alginate consisting of only M-blocks or (iii) an alginate consisting of MG-blocks. In some embodiments, the alginate is an alginate consisting of G-blocks. In some embodiments, the alginate is an alginate consisting of only M-blocks. In some embodiments, the alginate is an alginate consisting of MG-blocks. In some embodiments, the alginate comprises guluronic acid in an amount greater than 50% by weight of said alginate. In some embodiments, the alginate comprises guluronic acid in an amount less than 50% by weight of said alginate.

In some embodiments, the alginate comprises at least one cell adhesion peptide covalently linked thereto. In some embodiments, the alginate comprises at least one cell adhesion peptide covalently linked thereto, wherein the cell adhesion peptide comprises RGD. In some embodiments, cell adhesion peptides comprise RGD, YIGSR (SEQ ID NO:1), IKVAV (SEQ ID NO:2), REDV (SEQ ID NO:3), DGEA (SEQ ID NO:4), VGVAPG (SEQ ID NO:5), GRGDS (SEQ ID NO:6), LDV, RGDV (SEQ ID NO:7), PDSGR (SEQ ID NO:8), RYVVLPR (SEQ ID NO:9), LGTIPG (SEQ ID NO:10), LAG, RGDS (SEQ ID NO:11), RGDF (SEQ ID NO:12), HHLGGALQAGDV (SEQ ID NO:13), VTCG (SEQ ID NO:14), SDGD (SEQ ID NO:15), GREDVY (SEQ ID NO:16), GRGDY (SEQ ID NO:17), GRGDSP (SEQ ID NO:18), VAPG (SEQ ID NO:19), GGGGRGDSP (SEQ ID NO:20) and GGGGRGDY (SEQ ID NO:21) and FTLCFD (SEQ ID NO:22). Biologically active molecules for cell adhesion or other cellular interaction may include EGF, VEGF, b-FGF, FGF, TGF, TGF-β or proteoglycans. Cell attachment peptides comprising RGD may be in some embodiments, 3, 4, 5, 6, 7, 8, 9 or 10 amino acids in length.. Suitable cell adhesion peptides comprising RGD include, but are not limited, to Novatach RGD (NovaMatrix, FMC BioPolymer, Oslo, Norway) and those disclosed in U.S. Patent No. 6,642,363, which is hereby incorporated by reference in its entirety. Peptide synthesis services are available from numerous companies, including Commonwealth Biotechnologies, Inc. of Richmond, Virginia, USA. Chemical techniques for coupling peptides to the alginate backbones may be found in U.S. Patent 6,642,363.

In some embodiments, the hydrogel further comprises equal to or less than 2%, 1.5%, 1%, or 0.5% of an alginate with a molecular weight of equal to or greater than 75,000 Daltons. In some embodiments, the hydrogel further comprises equal to or less than 2% of an alginate with a molecular weight of equal to or greater than 75,000 Daltons. In some embodiments, the hydrogel further comprises equal to or less than 1% of an alginate with a molecular weight of equal to or greater than 75,000 Daltons.

In some embodiments, at least 90% of said hydrogel is not dissolved in physiological fluid in less than 90 days. In some embodiments, at least 90% of said hydrogel is not dissolved in physiological fluid in than 80, less than 70, less than 60, less than 50, less than 40, or less than 30 days. In some embodiments, at least 90% of said hydrogel is not dissolved in physiological fluid in less than 120 days. In some embodiments, at least 80% of said hydrogel is not dissolved in physiological fluid in less than 30, less than 90 or less than 120 days. In some embodiments, at least 70% of said hydrogel is not dissolved in physiological fluid in less than 30, less than 90 or less than 120 days.

In some embodiments, the hydrogel is at least 90% dissolved in physiological fluid not less than 3 days.

In some embodiments, the hydrogel further comprises at least one of a polysaccharide. In some embodiments, the polysaccharide comprises at least one of hyaluronic acid or chitosan.

In some embodiments, the hydrogel further comprises at least one of calcium, strontium or barium ionically bound to said alginate. In some embodiments, the ionically bound barium in the hydrogel is used as a radio-opaque material.

In some embodiments, the hydrogel is sterilized. In some embodiments, the alginate of the hydrogel is sterilized. In some embodiments, the hydrogel is sterilized by gamma radiation. In some embodiments, the alginate of the hydrogel is sterilized by gamma radiation. In some embodiments, the sterilization comprises γ-irradiation, E-beam, ethylene oxide, autoclaving or contacting the hydrogel with alcohol prior to addition of the liquid component or contacting with NOx gases, hydrogen gas plasma sterilization.

In some embodiments, said alginate has an endotoxin level equal to or less than 1,500 EU/g, 1,000 EU/g, 500 EU/g, 150 EU/g, 100 EU/g, 75 EU/g, 50 EU/g, or 35 EU/g. In some embodiments, said hydrogel has an endotoxin level equal to or less than 1,500 EU/g, 1,000 EU/g, 500 EU/g, 150 EU/g, 100 EU/g, 75 EU/g, 50 EU/g, or 35 EU/g.

In each of the embodiments described herein, the hydrogel may further comprises at least one of cells, pharmaceutically active agents, nutritional active agents, tissue, drugs, food, cosmetic agents, or radioactive isotopes. In some embodiments, the cells comprise islet cells, kerotinocytes, hepatocytes, nephrons, chondrocytes, myoblasts, fibroblasts, or neurons; and said pharmaceutically active agents comprise antibiotics, cancer chemotherapeutics, morphine, growth factors and anti-infective agents. In some embodiments, the hydrogel further comprises cells. In some embodiments, the hydrogel further comprises cells selected from the group consisting of islet cells, kerotinocytes, hepatocytes, nephrons, chondrocytes, myoblasts, fibroblasts, and neurons. In some embodiments, the cells comprise islet cells.

A wide variety of cells appropriate for use in accordance with the hydrogels described herein, as will be readily appreciated by one of skill in the art of cell implantation. Appropriate cells (autologous, allogeneic, xenogeneic) include, for example, hepatocytes, all types of stem cells (with the exception of embryonic stem cells), insulin producing cells including cells derived from stem cells of any origin (e.g., pancreatic islet cells, isolated pancreatic beta cells, insulinoma cells, etc (with the exception of embryonic stem cells)), endocrine hormone-producing cells (e.g., parathyroid, thyroid, adrenal, etc.) and any genetically engineered cells that secrete therapeutic agents, such as proteins or hormones for treating disease or other conditions, and genetically engineered cells that secrete diagnostic agents. In some embodiments, the hydrogel comprises pancreatic islet cells, hepatic cells, neural cells, vascular endothelial cells, thyroid cells, adrenal cells, thymic cells and ovarian cells. In some embodiments, the hydrogel comprises cells are selected from the group consisting of pancreatic islet cells, mesenchymal stem cells, parathyroid cells, and thyroid cells. In some embodiments, the hydrogel comprises cells are selected from the group consisting of pancreatic islet cells and parathyroid cells. In some embodiments, the hydrogel comprises pancreatic islet cells..In some embodiments, the hydrogel comprises tissue of any of the cells described herein.

In some embodiments, the cells comprise cells from a human or pig. In some embodiments, the cells comprise cells from a human, neonatal pig, or an adult pig. In some embodiments, the cells comprise cells from a human. In some embodiments, the cells comprise cells from a neonatal pig, or an adult pig. In some embodiments, the cells comprise cells from a neonatal pig. In some embodiments, the cells comprise cells from an adult pig. In some embodiments, the pancreatic islet cells comprise cells from a human or pig. In some embodiments, the pancreatic islet cells comprise cells from a human, neonatal pig, or an adult pig. In some embodiments, the pancreatic islet cells comprise cells from a human. In some embodiments, the pancreatic islet cells comprise cells from a neonatal pig, or an adult pig. In some embodiments, the pancreatic islet cells comprise cells from a neonatal pig. In some embodiments, the pancreatic islet cells comprise cells from an adult pig.

In some embodiments, the alginate has a molecular weight less than 50,000 Daltons and is present in an amount of at least 4% by weight of the gel. In some embodiments, the alginate has a molecular weight less than 50,000 Daltons and is present in an amount of at least 5% by weight of the gel. In some embodiments, the alginate has a molecular weight less than 50,000 Daltons and is present in an amount of at least 6% by weight of the gel. In some embodiments, the alginate has a molecular weight less than 50,000 Daltons and is present in an amount of at least 7% by weight of the gel. In some embodiments, the alginate has a molecular weight less than 50,000 Daltons and is present in an amount of at least 8% by weight of the gel.

In some embodiments, the alginate has a molecular weight less than 40,000 Daltons, wherein said alginate is present in an amount of at least 4% by weight of the gel. In some embodiments, the alginate has a molecular weight less than 40,000 Daltons and is present in an amount of at least 5% by weight of the gel. In some embodiments, the alginate has a molecular weight less than 40,000 Daltons and is present in an amount of at least 6% by weight of the gel. In some embodiments, the alginate has a molecular weight less than 40,000 Daltons and is present in an amount of at least 7% by weight of the gel. In some embodiments, the alginate has a molecular weight less than 40,000 Daltons and is present in an amount of at least 8% by weight of the gel.

In some embodiments, the alginate has a molecular weight less than 30,000 Daltons and is present in an amount of at least 4% by weight of the gel. In some embodiments, the alginate has a molecular weight less than 30,000 Daltons and is present in an amount of at least 5% by weight of the gel. In some embodiments, the alginate has a molecular weight less than 30,000 Daltons and is present in an amount of at least 6% by weight of the gel. In some embodiments, the alginate has a molecular weight less than 30,000 Daltons and is present in an amount of at least 7% by weight of the gel. In some embodiments, the alginate has a molecular weight less than 30,000 Daltons and is present in an amount of at least 8% by weight of the gel.

In some embodiments, the alginate has a molecular weight less than 20,000 Daltons and is present in an amount of at least 4% by weight of the gel. In some embodiments, the alginate has a molecular weight less than 20,000 Daltons and is present in an amount of at least 5% by weight of the gel. In some embodiments, the alginate has a molecular weight less than 20,000 Daltons and is present in an amount of at least 6% by weight of the gel. In some embodiments, the alginate has a molecular weight less than 20,000 Daltons and is present in an amount of at least 7% by weight of the gel. In some embodiments, the alginate has a molecular weight less than 20,000 Daltons and is present in an amount of at least 8% by weight of the gel.

In some embodiments, the alginate has a molecular weight less than 50,000 Daltons and is present in an amount of at least 4% by weight of the gel; wherein said alginate has an endotoxin level equal to or less than 1,000 EU/g. In some embodiments, the alginate has a molecular weight less than 50,000 Daltons and is present in an amount of at least 5% by weight of the gel; wherein said alginate has an endotoxin level equal to or less than 1,000 EU/g. In some embodiments, the alginate has a molecular weight less than 50,000 Daltons and is present in an amount of at least 6% by weight of the gel; wherein said alginate has an endotoxin level equal to or less than 1,000 EU/g. In some embodiments, the alginate has a molecular weight less than 50,000 Daltons and is present in an amount of at least 7% by weight of the gel; wherein said alginate has an endotoxin level equal to or less than 1,000 EU/g. In some embodiments, the alginate has a molecular weight less than 50,000 Daltons and is present in an amount of at least 8% by weight of the gel; wherein said alginate has an endotoxin level equal to or less than 1,000 EU/g.

In some embodiments, the hydrogel comprises an alginate having a molecular weight less than 40,000 Daltons, wherein said alginate is present in an amount of at least 4% by weight of the gel; wherein said alginate has an endotoxin level equal to or less than 1,000 EU/g. In some embodiments, the alginate has a molecular weight less than 40,000 Daltons and is present in an amount of at least 5% by weight of the gel; wherein said alginate has an endotoxin level equal to or less than 1,000 EU/g. In some embodiments, the alginate has a molecular weight less than 40,000 Daltons and is present in an amount of at least 6% by weight of the gel; wherein said alginate has an endotoxin level equal to or less than 1,000 EU/g. In some embodiments, the alginate has a molecular weight less than 40,000 Daltons and is present in an amount of at least 7% by weight of the gel; wherein said alginate has an endotoxin level equal to or less than 1,000 EU/g. In some embodiments, the alginate has a molecular weight less than 40,000 Daltons and is present in an amount of at least 8% by weight of the gel; wherein said alginate has an endotoxin level equal to or less than 1,000 EU/g.

In some embodiments, the hydrogel comprises an alginate having a molecular weight less than 30,000 Daltons, wherein said alginate is present in an amount of at least 4% by weight of the gel; wherein said alginate has an endotoxin level equal to or less than 1,000 EU/g. In some embodiments, the alginate has a molecular weight less than 30,000 Daltons and is present in an amount of at least 5% by weight of the gel; wherein said alginate has an endotoxin level equal to or less than 1,000 EU/g. In some embodiments, the alginate has a molecular weight less than 30,000 Daltons and is present in an amount of at least 6% by weight of the gel; wherein said alginate has an endotoxin level equal to or less than 1,000 EU/g. In some embodiments, the alginate has a molecular weight less than 30,000 Daltons and is present in an amount of at least 7% by weight of the gel; wherein said alginate has an endotoxin level equal to or less than 1,000 EU/g. In some embodiments, the alginate has a molecular weight less than 30,000 Daltons and is present in an amount of at least 8% by weight of the gel; wherein said alginate has an endotoxin level equal to or less than 1,000 EU/g.

In some embodiments, the hydrogel comprises an alginate having a molecular weight less than 20,000 Daltons, wherein said alginate is present in an amount of at least 4% by weight of the gel; wherein said alginate has an endotoxin level equal to or less than 1,000 EU/g. In some embodiments, the alginate has a molecular weight less than 20,000 Daltons and is present in an amount of at least 5% by weight of the gel; wherein said alginate has an endotoxin level equal to or less than 1,000 EU/g. In some embodiments, the alginate has a molecular weight less than 20,000 Daltons and is present in an amount of at least 6% by weight of the gel; wherein said alginate has an endotoxin level equal to or less than 1,000 EU/g. In some embodiments, the alginate has a molecular weight less than 20,000 Daltons and is present in an amount of at least 7% by weight of the gel; wherein said alginate has an endotoxin level equal to or less than 1,000 EU/g. In some embodiments, the alginate has a molecular weight less than 20,000 Daltons and is present in an amount of at least 8% by weight of the gel; wherein said alginate has an endotoxin level equal to or less than 1,000 EU/g.

In each of the embodiments described in the previous eight paragraphs, or combination thereof, the hydrogel may further comprises at least one of cells, pharmaceutically active agents, nutritional active agents, tissue, drugs, food, cosmetic agents, or radioactive isotopes. In some embodiments, the cells comprise islet cells, kerotinocytes, hepatocytes, nephrons, chondrocytes, myoblasts, fibroblasts, or neurons; and said pharmaceutically active agents comprise antibiotics, cancer chemotherapeutics, morphine, growth factors and anti-infective agents. In some embodiments, the hydrogel further comprises cells. In some embodiments, the hydrogel further comprises cells selected from the group consisting of islet cells, kerotinocytes, hepatocytes, nephrons, chondrocytes, myoblasts, fibroblasts, and neurons. In some embodiments, the cells comprise islet cells.

In a fifth aspect, the present invention further provides hydrogels having a mixture of low and high molecular weight alginate made by a self-gelling process. The delay in the self-gelling process may be used to allow for the injection of solutions into the body and/or to mix cells or other biomaterial into the gel matrix prior to the gel forming. Such hydrogels are useful as biodegradable materials for use in various medical applications. According, the hydrogels are formed by a method comprising:
a) forming a dispersion by mixing i) a solution comprising a soluble alginate with an insoluble gel particles or ii) immediately soluble alginate, insoluble gel particles and a solvent, and
b) dispensing the dispersion whereby the dispersion forms a hydrogel; wherein said soluble alginate or immediately soluble alginate has a molecular weight less than 75,000 Daltons, wherein the soluble alginate is present in an amount of at least 2.5% by weight of the hydrogel. As used herein, the term "insoluble gel particles" refers to dried particles of gel formed by adding gelling ions to an alginate polymer having a molecular weight above 75,000 Daltons. In some embodiments, the insoluble gel particles comprises an alginate polymer having a molecular weight above 100,000 Daltons, above 150,000 Daltons, or above 200,000 Daltons. The insoluble gel particles can be prepared as described in U.S. Patent Publication 2006/0159823, published July 20, 2006, (refers to the insoluble gel particles as "insoluble alginate/gelling ion particles").

In some embodiments, the soluble alginate is freeze dried or otherwise desiccated. Freeze dried soluble alginate is "immediately soluble." "Immediately soluble" alginate is soluble in water in less than one minute, preferably less than 30 seconds, more preferably less than 15 seconds. The molecular weights and concentrations of alginate described above for the other hydrogel embodiments (and combinations thereof) can be used for the soluble alginate of the self-gelling hydrogels.

In some embodiments, the soluble alginate or immediately soluble alginate each independently has a molecular weight of less than 50,000, 40,000, 30,000, 20,000, or about 10,000 Daltons. In some embodiments, the soluble alginate soluble alginate or immediately soluble alginate is present in an amount of at least 3%, 4%, 5%, 6%, 7%, 8%, or 9% by weight of the gel.

In some embodiments, the soluble alginate or immediately soluble alginate each independently has a molecular weight less than 50,000 Daltons and is present in an amount of at least 4% by weight of the gel. In some embodiments, the soluble alginate or immediately soluble alginate each independently has a molecular weight less than 50,000 Daltons and is present in an amount of at least 5% by weight of the gel. In some embodiments, the soluble alginate or immediately soluble alginate each independently has a molecular weight less than 50,000 Daltons and is present in an amount of at least 6% by weight of the gel. In some embodiments, the soluble alginate or immediately soluble alginate each independently has a molecular weight less than 50,000 Daltons and is present in an amount of at least 7% by weight of the gel. In some embodiments, the soluble alginate or immediately soluble alginate each independently has a molecular weight less than 50,000 Daltons and is present in an amount of at least 8% by weight of the gel.

In some embodiments, the soluble alginate or immediately soluble alginate each independently has a molecular weight less than 40,000 Daltons and is present in an amount of at least 4% by weight of the gel. In some embodiments, the soluble alginate or immediately soluble alginate each independently has a molecular weight less than 40,000 Daltons and is present in an amount of at least 5% by weight of the gel. In some embodiments, the soluble alginate or immediately soluble alginate each independently has a molecular weight less than 40,000 Daltons and is present in an amount of at least 6% by weight of the gel. In some embodiments, the soluble alginate or immediately soluble alginate each independently has a molecular weight less than 450,000 Daltons and is present in an amount of at least 7% by weight of the gel. In some embodiments, the soluble alginate or immediately soluble alginate each independently has a molecular weight less than 40,000 Daltons and is present in an amount of at least 8% by weight of the gel.

In some embodiments, the soluble alginate or immediately soluble alginate each independently has a molecular weight less than 30,000 Daltons and is present in an amount of at least 4% by weight of the gel. In some embodiments, the soluble alginate or immediately soluble alginate each independently has a molecular weight less than 30,000 Daltons and is present in an amount of at least 5% by weight of the gel. In some embodiments, the soluble alginate or immediately soluble alginate each independently has a molecular weight less than 30,000 Daltons and is present in an amount of at least 6% by weight of the gel. In some embodiments, the soluble alginate or immediately soluble alginate each independently has a molecular weight less than 30,000 Daltons and is present in an amount of at least 7% by weight of the gel. In some embodiments, the soluble alginate or immediately soluble alginate each independently has a molecular weight less than 30,000 Daltons and is present in an amount of at least 8% by weight of the gel.

In some embodiments, the soluble alginate or immediately soluble alginate each independently has a molecular weight less than 20,000 Daltons and is present in an amount of at least 4% by weight of the gel. In some embodiments, the soluble alginate or immediately soluble alginate each independently has a molecular weight less than 20,000 Daltons and is present in an amount of at least 5% by weight of the gel. In some embodiments, the soluble alginate or immediately soluble alginate each independently has a molecular weight less than 20,000 Daltons and is present in an amount of at least 6% by weight of the gel. In some embodiments, the soluble alginate or immediately soluble alginate each independently has a molecular weight less than 20,000 Daltons and is present in an amount of at least 7% by weight of the gel. In some embodiments, the soluble alginate or immediately soluble alginate each independently has a molecular weight less than 20,000 Daltons and is present in an amount of at least 8% by weight of the gel.

In some embodiments, the soluble alginate or immediately soluble alginate each independently has a molecular weight less than 50,000 Daltons and is present in an amount of at least 4% by weight of the gel; wherein said soluble alginate or immediately soluble alginate each independently has an endotoxin level equal to or less than 1,000 EU/g. In some embodiments, the soluble alginate or immediately soluble alginate each independently has a molecular weight less than 50,000 Daltons and is present in an amount of at least 5% by weight of the gel; wherein said soluble alginate or immediately soluble alginate each independently has an endotoxin level equal to or less than 1,000 EU/g. In some embodiments, the soluble alginate or immediately soluble alginate each independently has a molecular weight less than 50,000 Daltons and is present in an amount of at least 6% by weight of the gel; wherein said soluble alginate or immediately soluble alginate each independently has an endotoxin level equal to or less than 1,000 EU/g. In some embodiments, the soluble alginate or immediately soluble alginate each independently has a molecular weight less than 50,000 Daltons and is present in an amount of at least 7% by weight of the gel; wherein said soluble alginate or immediately soluble alginate each independently has an endotoxin level equal to or less than 1,000 EU/g. In some embodiments, the soluble alginate or immediately soluble alginate each independently has a molecular weight less than 50,000 Daltons and is present in an amount of at least 8% by weight of the gel; wherein said soluble alginate or immediately soluble alginate each independently has an endotoxin level equal to or less than 1,000 EU/g.

In some embodiments, the hydrogel comprises an soluble alginate or immediately soluble alginate each has a molecular weight less than 30,000 Daltons and is present in an amount of at least 4% by weight of the gel; wherein said soluble alginate or immediately soluble alginate each independently has an endotoxin level equal to or less than 1,000 EU/g. In some embodiments, the soluble alginate or immediately soluble alginate each independently has a molecular weight less than 30,000 Daltons and is present in an amount of at least 5% by weight of the gel; wherein said soluble alginate or immediately soluble alginate each independently has an endotoxin level equal to or less than 1,000 EU/g. In some embodiments, the soluble alginate or immediately soluble alginate each independently has a molecular weight less than 30,000 Daltons and is present in an amount of at least 6% by weight of the gel; wherein said soluble alginate or immediately soluble alginate each independently has an endotoxin level equal to or less than 1,000 EU/g. In some embodiments, the soluble alginate or immediately soluble alginate each independently has a molecular weight less than 30,000 Daltons and is present in an amount of at least 7% by weight of the gel; wherein said soluble alginate or immediately soluble alginate each independently has an endotoxin level equal to or less than 1,000 EU/g. In some embodiments, the soluble alginate or immediately soluble alginate each independently has a molecular weight less than 30,000 Daltons and is present in an amount of at least 8% by weight of the gel; wherein said soluble alginate or immediately soluble alginate each independently has an endotoxin level equal to or less than 1,000 EU/g.

In some of the embodiments, the hydrogel produced by the self-gelling method may further comprises at least one of cells, pharmaceutically active agents, nutritional active agents, tissue, drugs, food, cosmetic agents, or radioactive isotopes. In some embodiments, the cells comprise islet cells, kerotinocytes, hepatocytes, nephrons, chondrocytes, myoblasts, fibroblasts, or neurons; and said pharmaceutically active agents comprise antibiotics, cancer chemotherapcutics, morphine, growth factors and anti-infective agents. In some embodiments, the hydrogel further comprises cells. In some embodiments, the hydrogel further comprises cells selected from the group consisting of islet cells, kerotinocytes, hepatocytes, nephrons, chondrocytes, myoblasts, fibroblasts, and neurons. In some embodiments, the cells comprise islet cells.

In some embodiments, the hydrogel made via the self-gelling method has an endotoxin level of less than 1500, 100, 500, 100, 50, 25, or 10 EU/g. In some embodiments, said hydrogel is at least 90% dissolved in physiological fluid in not less than 3 days. In some embodiments, said hydrogel is at least 90% dissolved in physiological fluid less than 6 weeks. In some embodiments, said hydrogel is at least 90% dissolved in physiological fluid less than 3 weeks. In some embodiments, said hydrogel is at least 90% dissolved in physiological fluid less than 1 week.

In some embodiments, the soluble alginate or immediately soluble alginate comprises at least one of: (i) an alginate consisting of G-blocks, (ii) an alginate consisting of only M-blocks or (iii) an alginate consisting of MG-blocks. In some embodiments, the soluble alginate or immediately soluble alginate is an alginate consisting of G-blocks. In some embodiments, the soluble alginate or immediately soluble alginate comprises at least one cell adhesion peptide covalently linked thereto.

In some embodiments, said soluble alginate, immediately soluble alginate, and alginate of the insoluble gelled alginate particles each has an endotoxin level equal to or less than 1,500 EU/g, 1,000 EU/g, 500 EU/g, 150 EU/g, 100 EU/g, 75 EU/g, 50 EU/g, or 35 EU/g. In some embodiments, said hydrogel has an endotoxin level equal to or less than 1,500 EU/g, 1,000 EU/g, 500 EU/g, 150 EU/g, 100 EU/g, 75 EU/g, 50 EU/g, or 35 EU/g.

In a sixth aspect, the present invention further provides beads comprising the embodiments of the hydrogels described herein, as well as any combination of those embodiments.

In a seventh aspect, the present invention provides beads comprising a hydrogel comprising an alginate having a molecular weight of less than 75,000 Daltons, wherein said alginate is present in an amount of at least 4% by weight of the gel.

In some embodiments, the alginate each independently has a molecular weight of less than 50,000, 40,000, 30,000, 20,000, or about 10,000 Daltons. In some embodiments, the alginate is present in an amount of at least 3%, 4%, 5%, 6%, 7%, 8%, or 9% by weight of the gel.

In some embodiments, the alginate has a molecular weight less than 50,000 Daltons and is present in an amount of at least 4% by weight of the gel. In some embodiments, the alginate has a molecular weight less than 50,000 Daltons and is present in an amount of at least 5% by weight of the gel. In some embodiments, the alginate has a molecular weight less than 50,000 Daltons and is present in an amount of at least 6% by weight of the gel. In some embodiments, the alginate has a molecular weight less than 50,000 Daltons and is present in an amount of at least 7% by weight of the gel. In some embodiments, the alginate has a molecular weight less than 50,000 Daltons and is present in an amount of at least 8% by weight of the gel.

In some embodiments, the alginate has a molecular weight less than 40,000 Daltons, wherein said alginate is present in an amount of at least 4% by weight of the gel. In some embodiments, the alginate has a molecular weight less than 40,000 Daltons and is present in an amount of at least 5% by weight of the gel. In some embodiments, the alginate has a molecular weight less than 40,000 Daltons and is present in an amount of at least 6% by weight of the gel. In some embodiments, the alginate has a molecular weight less than 40,000 Daltons and is present in an amount of at least 7% by weight of the gel. In some embodiments, the alginate has a molecular weight less than 40,000 Daltons and is present in an amount of at least 8% by weight of the gel.

In some embodiments, the alginate has a molecular weight less than 30,000 Daltons and is present in an amount of at least 4% by weight of the gel. In some embodiments, the alginate has a molecular weight less than 30,000 Daltons and is present in an amount of at least 5% by weight of the gel. In some embodiments, the alginate has a molecular weight less than 30,000 Daltons and is present in an amount of at least 6% by weight of the gel. In some embodiments, the alginate has a molecular weight less than 30,000 Daltons and is present in an amount of at least 7% by weight of the gel. In some embodiments, the alginate has a molecular weight less than 30,000 Daltons and is present in an amount of at least 8% by weight of the gel.

In some embodiments, the alginate has a molecular weight less than 20,000 Daltons and is present in an amount of at least 4% by weight of the gel. In some embodiments, the alginate has a molecular weight less than 20,000 Daltons and is present in an amount of at least 5% by weight of the gel. In some embodiments, the alginate has a molecular weight less than 20,000 Daltons and is present in an amount of at least 6% by weight of the gel. In some embodiments, the alginate has a molecular weight less than 20,000 Daltons and is present in an amount of at least 7% by weight of the gel. In some embodiments, the alginate has a molecular weight less than 20,000 Daltons and is present in an amount of at least 8% by weight of the gel.

In some embodiments, the alginate has a molecular weight less than 50,000 Daltons and is present in an amount of at least 4% by weight of the gel; wherein said alginate has an endotoxin level equal to or less than 1,000 EU/g. In some embodiments, the alginate has a molecular weight less than 50,000 Daltons and is present in an amount of at least 5% by weight of the gel; wherein said alginate has an endotoxin level equal to or less than 1,000 EU/g. In some embodiments, the alginate has a molecular weight less than 50,000 Daltons and is present in an amount of at least 6% by weight of the gel; wherein said alginate has an endotoxin level equal to or less than 1,000 EU/g. In some embodiments, the alginate has a molecular weight less than 50,000 Daltons and is present in an amount of at least 7% by weight of the gel; wherein said alginate has an endotoxin level equal to or less than 1,000 EU/g. In some embodiments, the alginate has a molecular weight less than 50,000 Daltons and is present in an amount of at least 8% by weight of the gel; wherein said alginate has an endotoxin level equal to or less than 1,000 EU/g.

In some embodiments, the hydrogel comprises an alginate having a molecular weight less than 40,000 Daltons, wherein said alginate is present in an amount of at least 4% by weight of the gel; wherein said alginate has an endotoxin level equal to or less than 1,000 EU/g. In some embodiments, the alginate has a molecular weight less than 40,000 Daltons and is present in an amount of at least 5% by weight of the gel; wherein said alginate has an endotoxin level equal to or less than 1,000 EU/g. In some embodiments, the alginate has a molecular weight less than 40,000 Daltons and is present in an amount of at least 6% by weight of the gel; wherein said alginate has an endotoxin level equal to or less than 1,000 EU/g. In some embodiments, the alginate has a molecular weight less than 40,000 Daltons and is present in an amount of at least 7% by weight of the gel; wherein said alginate has an endotoxin level equal to or less than 1,000 EU/g. In some embodiments, the alginate has a molecular weight less than 40,000 Daltons and is present in an amount of at least 8% by weight of the gel; wherein said alginate has an endotoxin level equal to or less than 1,000 EU/g.

In some embodiments, the hydrogel comprises an alginate having a molecular weight less than 30,000 Daltons, wherein said alginate is present in an amount of at least 4% by weight of the gel; wherein said alginate has an endotoxin level equal to or less than 1,000 EU/g. In some embodiments, the alginate has a molecular weight less than 30,000 Daltons and is present in an amount of at least 5% by weight of the gel; wherein said alginate has an endotoxin level equal to or less than 1,000 EU/g. In some embodiments, the alginate has a molecular weight less than 30,000 Daltons and is present in an amount of at least 6% by weight of the gel; wherein said alginate has an endotoxin level equal to or less than 1,000 EU/g. In some embodiments, the alginate has a molecular weight less than 30,000 Daltons and is present in an amount of at least 7% by weight of the gel; wherein said alginate has an endotoxin level equal to or less than 1,000 EU/g. In some embodiments, the alginate has a molecular weight less than 30,000 Daltons and is present in an amount of at least 8% by weight of the gel; wherein said alginate has an endotoxin level equal to or less than 1,000 EU/g.

In some embodiments, the hydrogel comprises an alginate having a molecular weight less than 20,000 Daltons, wherein said alginate is present in an amount of at least 4% by weight of the gel; wherein said alginate has an endotoxin level equal to or less than 1,000 EU/g. In some embodiments, the alginate has a molecular weight less than 20,000 Daltons and is present in an amount of at least 5% by weight of the gel; wherein said alginate has an endotoxin level equal to or less than 1,000 EU/g. In some embodiments, the alginate has a molecular weight less than 20,000 Daltons and is present in an amount of at least 6% by weight of the gel; wherein said alginate has an endotoxin level equal to or less than 1,000 EU/g. In some embodiments, the alginate has a molecular weight less than 20,000 Daltons and is present in an amount of at least 7% by weight of the gel; wherein said alginate has an endotoxin level equal to or less than 1,000 EU/g. In some embodiments, the alginate has a molecular weight less than 20,000 Daltons and is present in an amount of at least 8% by weight of the gel; wherein said alginate has an endotoxin level equal to or less than 1,000 EU/g.

In some embodiments of the previous ten paragraphs, or combination thereof, the hydrogel may further comprises at least one of cells, pharmaceutically active agents, nutritional active agents, tissue, drugs, food, cosmetic agents, or radioactive isotopes. In some embodiments, the cells comprise islet cells, kerotinocytes, hepatocytes, nephrons, chondrocytes, myoblasts, fibroblasts, or neurons; and said pharmaceutically active agents comprise antibiotics, cancer chemotherapeutics, morphine, growth factors and anti-infective agents. In some embodiments, the hydrogel further comprises cells. In some embodiments, the hydrogel further comprises cells selected from the group consisting of islet cells, kerotinocytes, hepatocytes, nephrons, chondrocytes, myoblasts, fibroblasts, and neurons. In some embodiments, the cells comprise islet cells.

In some embodiments of the previous eleven paragraphs, or combination thereof, the alginate comprises at least one of: (i) an alginate consisting of G-blocks, (ii) an alginate consisting of only M-blocks or (iii) an alginate consisting of MG-blocks. In some embodiments, the alginate is an alginate consisting of G-blocks. In some embodiments, the alginate comprises at least one cell adhesion peptide covalently linked thereto.

### Low Molecular Weight Alginates

Low molecular alginates suitable for use in the various aspects of present invention can be purchased or made by methods known in the art. Suitable alginates include PRONOVA UP VLVM and VLVM (NovaMatrix, FMC Biopolymer AS, Oslo, Norway). Suitable routes of synthesis for the low molecular weight alginates include, but are not limited to acid hydrolysis at elevated temperatures, or thermal depolymerisation, which induces degradation of the starting alginate. Particular molecular weight ranges can be produced by modifying the reaction time and temperatures. Suitable non-limiting preparations are presented below.

Preparation 1. 3.0 kg M-rich alginate was dissolved in 300 kg of purified water. The pH was adjusted to 4.3 +/- 0.1. Acid hydrolysis was performed for 18.5 hours, at temperature of 73 degrees C (start) to 62 degrees C (end). Sodium hydroxide was added to solution until pH 7.0. The product was dried (spray drying). The pH and viscosity of the product was 6.5 and 3.9 mPas, respectively.

Preparation 2. Protanal LFR 5/60 was degraded using hot steam. This product was subsequently further depolymerized using acid hydrolysis in solution (pH 4.3) at 80 degrees Celsius to produce six fractions as characterized in the table below.

| **Sample** | **Intrinsic Viscosity [ml/g]** | **Mw [g/mol]*** | **Mw [g/mol] truncated** | **Amount [mg]** |
|---|---|---|---|---|
| 0 | 161 | 30127 | 30000 | 56 |
| 3 | 133 | 24532 | 25000 | 263 |
| 4 | 106 | 19221 | 19000 | 304 |
| 5 | 70 | 12303 | 12000 | 335 |
| 6 | 41 | 6922 | 6900 | 176 |
| 7 | 28 | 4593 | 4600 | 3855 |
| | | | | |
| K= | 0.011 | | | |
| a= | 0.93 | | | |

Preparation 3. Thermal depolymerisation was conducted by thermal depolymerisation of a dry powder of Protanal LFR 5/60 at a temperature of 80 degrees C for a total treatment time of 305 to 385 hours.

Various fractionation techniques are also known for the production of low molecular weight alginates with a high content of guluronic aid blocks ("G-blocks"), mannuronate blocks ("M-blocks"), or MG blocks. For example, suitable non-limiting methods for forming such alginates are disclosed in U.S. Patent No. 6,121,441, filed May 8, 1998, and U.S. Patent No. 6,747,015, filed Oct. 2, 2001, each of which is hereby incorporated by reference in its entirety.

### Methods of Making the Hydrogels and Beads

The methods herein may be used to produce any of the embodiments of the hydrogels or beads hereinbefore described, including various combinations and subcombinations of the embodiments.

The present invention further provides a method of making a hydrogel, comprising the steps of preparing a solution of the alginate and adding said alginate solution to a solution comprising gelling cations to form said hydrogel.

The present invention further provides method of making a hydrogel, comprising mixing a compound containing gelling cations into a solution containing the alginate, wherein said cations are released at a desired rate in said alginate solution to form said hydrogel.

The present invention further provides a method of making hydrogels having a mixture of low and high molecular weight alginates made by a self-gelling process. According, the method comprises:
a) forming a dispersion by mixing i) a solution comprising a soluble alginate with an insoluble gel particles or ii) immediately soluble alginate, insoluble gel particles and a solvent, and
b) dispensing the dispersion whereby the dispersion forms a hydrogel; wherein said soluble alginate or immediately soluble alginate each independently has a molecular weight less than 75,000 Daltons, wherein the soluble alginate or immediately soluble alginate is present in an amount of at least 2.5% by weight of the hydrogel.

In some embodiments, the compound is at least one of calcium carbonate, calcium containing liposome, calcium sulfate, calcium phosphate, calcium lactate or calcium citrate. In some embodiments, the gelling cations are selected from the group consisting of strontium, barium, calcium, and combination thereof. In some embodiments, the gelling cations are calcium ions. In some embodiments, the gelling cations are barium ions. In some embodiments, the gelling cations are strontium ions. In some embodiments, the gelling cations are strontium and calcium ions. In some embodiments, the gelling cations are barium and calcium ions. In some embodiments, the gelling cations are barium and strontium ions.

A salt or combination of salts that provides the desired gelling cations or mixture of gel-forming ions may be used as the gel-forming ions. Suitable gel-forming ions for forming the gel or the coating include monovalent and polyvalent ions, preferably a divalent and/or a trivalent ions, or mixture of ions capable of forming a gel with the polysaccharide or which do not form a soluble salt with the alginate. For alginates, suitable polyvalent cations include, for example, calcium(2+), barium(2+), strontium(2+), iron(2+), zinc(2+), copper(2+), and aluminum(3+). Preferred cations are divalent metal cations, more preferably the calcium (2+) cation.

In some embodiments, the gelling cations are selected from the group consisting of strontium ions, barium ions, calcium ions, and combination thereof. In some embodiments, the gelling cations are selected from the group consisting of barium ions, calcium ions, and combination thereof. In some embodiments, the gelling cations are selected from the group consisting of strontium ions, calcium ions, and combination thereof. In some embodiments, the gel-forming ions in the hydrogel are strontium ions. In some embodiments, the gelling cations are calcium ions. In some embodiments, the gelling cations are barium ions. In some embodiments, the gelling cations are strontium ions.

The concentration of gelling cations needed to saturate 100% of the gelling sites of the alginate may be calculated, although the present invention includes hydrogels which are supersaturated or undersaturated by the gelling cations. For example, when sufficient gelling cations, such as calcium ion, are present to react with all available gelling sites (eg. the L-guluronic acid units in the case of alginate), the alginate is 100% saturated. The amount of cation required to completely saturate the gelling sites of alginate, for example, is considered to be 1 mole of divalent cation per 2 moles of L-guluronic acid in the alginate or 1 mole of trivalent cation per 3 moles of L-guluronic acid in the alginate when only a divalent cation or only a trivalent cation is used in the gelling. When a mixture of a divalent cation or cations and a trivalent cation or cations is used, the amounts required to saturate the alginate can be determined because a divalent cation occupies two gelling sites and a trivalent cation occupies three gelling sites.

The amount of divalent cation, such as calcium, required to react stoichiometrically with these G-blocks can be calculated for each alginate type by considering that two guluronic acid units plus one divalent cation are required to create one ionic crosslink. The amount of calcium required for stoichiometric saturation of a 1% sodium alginate solution is given in the following table:

| Seaweed Source | % G | mM Ca |
|---|---|---|
| *Laminaria hyperborea* (stem) | 70 | 14-16 |
| *Laminaria hyperborea* (leaf) | 54 | 11-13 |
| *Lessonia trabeculata* | 68 | 13-15 |
| *Macrocystis pyrifera* | 39 | 8-9 |

A list of various commercially available alginates, their properties, and their sources is found in Shapiro, U.S. Pat. No. 6,334,968, Table 1, column 16, line 49, to column 17, line 18, which is hereby incorporated herein by reference in its entirety. Mixtures or blends of alginates, for example alginates of different molecular weights and/or G content, may be used as the gel-forming polymer.

Complete saturation (100% saturation) of the gelling sites occurs when the composition contains 1 mole of divalent cation per 2 moles of L-guluronic acid units. For example, an about 15 mM solution of calcium ion is required to 100% saturate a 1% solution of sodium alginate extracted from the stems of *Laminaria hyperborea,* an about 12 mM calcium solution is required to 100% saturate a 1% solution of sodium alginate extracted from the leaves (fronds) of *Laminaria hyperborea,* and an about 14 mM solution of calcium ions is required to 100% saturate a 1% solution of sodium alginate extracted from *Lessonia trabeculata.* When using a sparingly soluble salt as the gel-forming ions, the extent of cross-linking can be controlled by controlling either the amount of gelling agent, for example, calcium carbonate, and/or the amount of solubilizing agent, for example a pH modifier such as glucono delta-lactone, present during gel formation.

In some embodiments, the solution of gelling cations used or formed during the processes has a concentration of about 5 mM to about 1000 mM. In some embodiments, the solution of gelling cations has a concentration of 10 mM to about 1000 mM. In some embodiments, the solution of gelling cations has a concentration of 20 mM to about 500 mM. In some embodiments, the solution of gelling cations has a concentration of 50 mM to about 100 mM. In some embodiments, the solution of gelling cations used or formed during the processes is at an isotonic concentration, wherein cells are added to the solution containing the alginate.

The present invention further provides a method of increasing the strength and stability of an alginate hydrogel towards chemical or physical stress comprising the step of preparing said hydrogel using the alginate as described in any of the embodiments above or combination thereof

### Methods of Using the Hydrogels or Beads, and Products Thereof

The present invention provides an implantable medical device comprising the hydrogel as described in any of the embodiments or combination thereof.

The present invention further provides a drug delivery formulation comprising the hydrogel as described in any of the embodiments or combination thereof.

The present invention further provides a contrast agent comprising the hydrogel as described in any of the embodiments or combination thereof.

The present invention further provides a method of implanting an implantable medical device in a patient in need thereof, comprising implanting one or more implantable medical devices as described in the embodiments herein, or any combination thereof, in said patient. In some embodiments, the hydrogel of the implantable medical device comprises at least one of cells, pharmaceutically active agents, nutritional active agents, tissue, drugs, food, cosmetic agents, or radioactive isotopes. In some embodiments, the hydrogel of the implantable medical device comprises cells. In some embodiments, the cells comprise islet cells, kerotinocytes, hepatocytes, nephrons, chondrocytes, myoblasts, fibroblasts, or neurons.

The present invention further provides a method of implanting a hydrogel bead in a patient in need thereof, comprising implanting one or more hydrogel beads as described in the embodiments herein, or any combination thereof, in said patient. In some embodiments, the hydrogel of hydrogel bead comprises at least one of cells, pharmaceutically active agents, nutritional active agents, tissue, drugs, food, cosmetic agents, or radioactive isotopes. In some embodiments, the hydrogel of hydrogel bead comprises cells. In some embodiments, the cells comprise islet cells, kerotinocytes, hepatocytes, nephrons, chondrocytes, myoblasts, fibroblasts, or neurons.

In some embodiments, cells immobilized in the medical device may be implanted into animals wherein the hydrogel acts as an immune barrier and prevents detection by the immune system thereby allowing the implantation of xenografts. In some embodiments, strontium may be used as gel-forming ions when animal cells are desired for implantation (xenografts), since when using this type of artificial organ, it is important that the cells do not grow out of the implanted device and become exposed to the immune system. In some embodiments, the device may also be useful to establish cell, tumor and tissue xenografts in animals for, for example, cancer research. Immobilization of multicellular aggregates, such as islets Langerhans, in the device allows said multicellular aggregates to be implanted into animals or humans without immune rejection and such implanted cell aggregates may then function as an artificial organ producing, for example, insulin. In some embodiments, the cells comprise islet cells, kerotinocytes, hepatocytes, nephrons, chondrocytes, myoblasts, fibroblasts, or neurons. In some embodiments, the cells comprise islet cells.

The technique described herein can be used for a variety of different cell types as described herein. The type of cell chosen will vary with the particular therapeutic use. The devices can be implanted by a variety of methods known to one of skill in the art. For example, the devices may be implanted by various methods known to those of skill in the art, such as subcutaneously, or surgically into various organs, muscles, tissues, or lumen of an organ. The hydrogels, beads, and implantable medical devices can be implanted into various tissues including, but not limited to, retroperitoneum, properitoneal space, mesentery, renal subcapular space, peritoneum, and intramuscular space.

In some embodiments, the one or more devices are implanted subcutaneously. In some embodiments, three to four devices are implanted into the patient.

In some embodiments, a therapeutically effective number of cells are implanted. The number of cells needed for the treatment of a specific disorder will vary depending the specific disorder(s) being treated, the size, age, and response pattern of the individual the severity of the disorder(s), the judgment of the attending clinician, the manner of administration, and the purpose of the administration, such as prophylaxis or therapy. The phrase "effective amount" refers to the number of cells that elicits the biological or medicinal response in a tissue, system, animal, individual, patient, or human that is being sought by a researcher, veterinarian, medical doctor or other clinician. The desired biological or medicinal response may include preventing the disorder in an individual (e.g., preventing the disorder in an individual that may be predisposed to the disorder, but does not yet experience or display the pathology or symptomatology of the disease). The desired biological or medicinal response may also include inhibiting the disorder in an individual that is experiencing or displaying the pathology or symptomatology of the disorder (i.e., arresting or slowing further development of the pathology and/or symptomatology). The desired biological or medicinal response may also include ameliorating the disorder in an individual that is experiencing or displaying the pathology or symptomatology of the disease (i.e., reversing the pathology or symptomatology).

One or more devices can be implanted in a patient to reach a therapeutically effective amount of cells. In addition, the number of cells may be divided between a first cell layer and a second cell layer if so desired. In some embodiments, the hydrogel comprises about 5,000 cells or more. In some embodiments, the hydrogel comprises about from about 5,000 to about 300,000 cells, about 5,000 to about 200,000 cells, about 5,000 to about 100,000 cells, about 10,000 to about 100,000 cells, about 5,000 to about 60,000 cells, about 10,000 to about 60,000 cells, about 20,000 to about 60,000, or about 20,000 to about 40,000 cells.

The entrapment of cells within the hydrogels may also be used in tissue engineering applications. For tissue engineering the growth of cells within or on 3-dimensional constructs is needed and therefore good biomaterials for such applications are needed. In particular, the hydrogels made via the self-gelling process described herein have shown degradation after subcutaneous and intramuscular implantation.

Injectable alginate/cell suspension systems may also be delivered to the defective or damaged tissue site even without surgical intervention. For such applications it may be critical to have a certain working time to shape the material before it gels. However, the gelation rate may also be required to be reasonable rapid so that a prolonged patient waiting time or problems with applying the gel/solution can be avoided. The hydrogels of the present invention allow for the inclusion of much larger concentrations of alginate, which may allow greater control over the degradation process. Further, the hydrogels of the self-gelling system may allow greater flexibility in administering the gels.

The present invention further provides a method of blocking blood vessels by using the beads or hydrogels described in any of the embodiments, or combination thereof, wherein the beads or hydrogels are resorbable. The present invention further provides an embolic therapeutic composition comprising the beads or hydrogels described in any of the embodiments, or combination thereof, wherein the beads or hydrogels are bioresorbable.

In particular, the hydrogels made by the self-gelling processes may also be particularly useful in embolization procedures in that it can be introduced to a blood vessel that is remotely accessible and dispensed as a liquid slurry to fully conform to interior or the blood vessel and more fully and effectively block it off relative to other types of closures such as sutures. The self-gelling solution may be dispensed in an amount sufficient to block off circulation upon formation of the hydrogel *in situ.* Further, the higher concentration of the low molecular weight polymer in the hydrogels may also allow for better strength of the blockage composition, or allow better control over the degradation process.

The present invention further provides a contrast agent or radio-opaque material comprising an alginate having a molecular weight less than 75,000 Daltons and barium ionically bound to said alginatc, wherein: said alginate is present in an amount of at least 2.5% by weight of the gel; and said barium in the hydrogel is used as a radio-opaque material. In some embodiments, said alginate has a molecular weight less than 50,000. In some embodiments, said alginate has a molecular weight less than 30,000. In some embodiments, said alginate is present in an amount of at least 4% by weight of the gel. In some embodiments, said alginate is present in an amount of at least 5% by weight of the gel. In some embodiments, said alginate is present in an amount of at least 6% by weight of the gel.

### EXAMPLES

The present invention will now be further described with reference to specific examples.

### Example 1: Increased stability of alginate beads in the presence of low MW alginates.

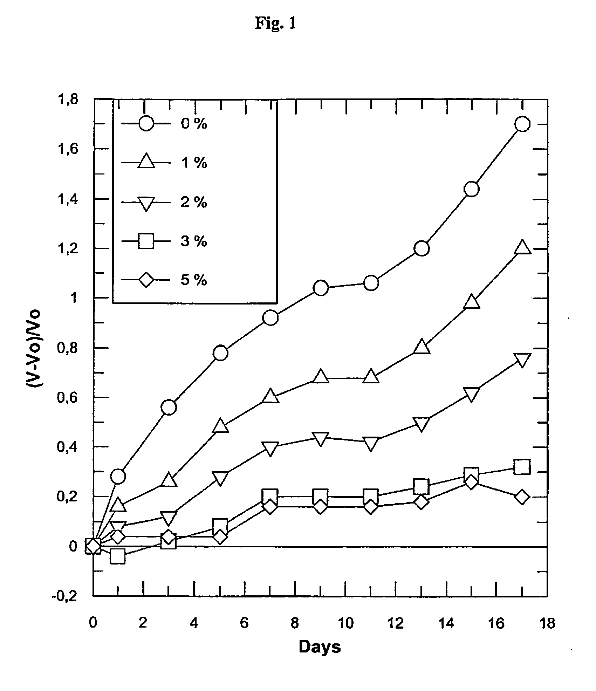

Alginate beads with a diameter of about 1 mm were made by dropping a solution of 2 % Protanal LF 10/60 (FMC Biopolymer, MW 100 000 - 170 000 g/mol) alone or in combination with 1-5 % of a highly degraded alginate from *Laminaria hyperborea* stem into a 50 mM solution of CaCl₂. The highly degraded alginate from *Laminaria Hyperborea* stem was degraded in solution by acid hydrolysis at elevated temperature (pH 4.3, 80 degrees Celsius, degradation time optimized with respect to desired MW) to a MW less than 25 kDa. The beads were kept in the CaCl₂ solution for at least 30 minutes. The highly degraded alginate did not have the ability to form gels on its own at the concentrations tested. 5 ml of the beads were transferred to measuring cylinders. The calcium chloride solution was removed and replaced with 25 ml 150 mM NaCl solution to mimic physiological salt concentrations. Every two days the cylinder volume with the beads filled was noted followed by an exchange of the NaCl solution. Figure 1 shows the relative swelling of the beads containing 0 to 5 % of the highly degraded alginate. The data clearly shows that higher alginate concentrations prevents destabilizing and swelling of the beads at physiological salt concentrations that occurs with time.

### Example 2. Increased mechanical strength of alginate beads in the presence of low MW alginates.

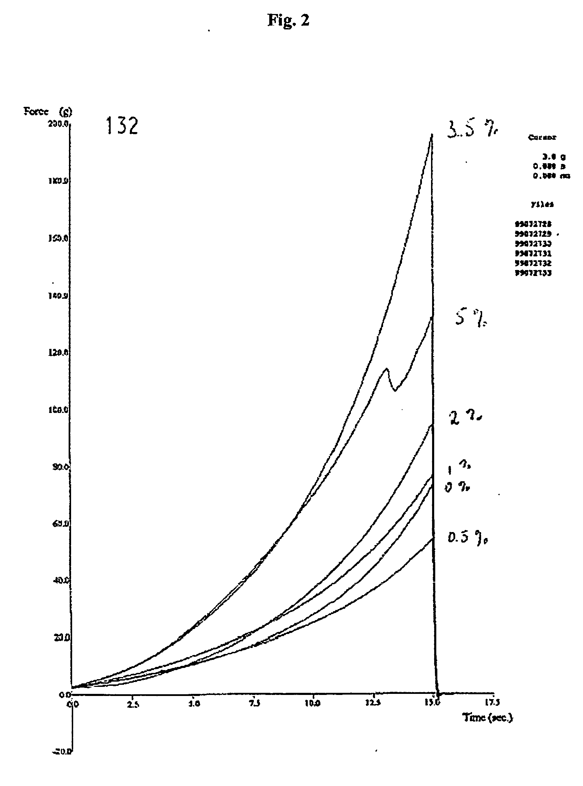

Alginate beads with a diameter of about 3 mm were made by dropping a solution of 2 % Protanal LF 10/60 (FMC Biopolymer, MW 100 000 - 170 000 g/mol) alone or in combination with 1-5 % of a highly degraded alginate from *Laminaria hyperborea* stem into a 50 mM solution of CaCl₂. The highly degraded alginate from *Laminaria Hyperborea* stem was degraded by acid hydrolysis in solution at elevated temperature (pH 4.3, 80 degrees Celsius, degradation time optimized with respect to desired MW) to a MW less than 25 kDa. The beads were kept in the CaCl₂ solution for at least 30 minutes. The highly degraded alginate did not have the ability to form gels on its own at the concentrations tested. The mechanical strength of individual beads was measured with a texture analyzer from Stable Microsystems using a flat probe. The rate of compression was 0.1 mm/s and the compression distance was set to 1.5 mm. The data (Figure 2) shows that the.mechanical strength increased with increasing amounts of highly degraded alginate up to about 3.5 %.

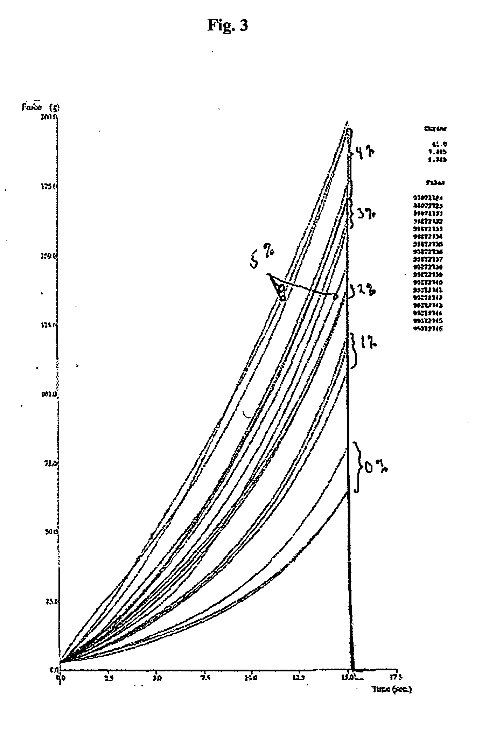

A similar experiment was also performed by making beads with a 2 % Protanal LF 10/60 alone or in combination with 1-5 % of a degraded alginate made from Protanal LFR 5/60 (FMC Biopolymer, MW 30 000 - 50 000 g/mol) (Figure 3). The experimental conditions were otherwise the same and the mechanical strength of individual beads was measured with the texture analyzer. In this case the data (Figure 3) showed that the mechanical strength did increase with increasing amounts of highly degraded alginate up to about 5 %.

### Example 3: Increased strength and stability of alginate beads using low MW alginates.

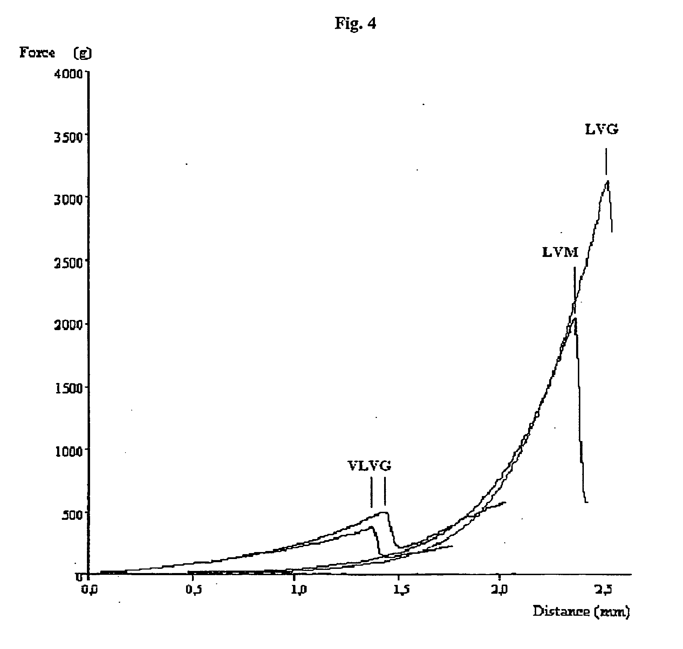

Alginate beads with a diameter of about 3 mm were made by dropping solutions of alginate into a 50 mM solution of CaCl₂. For all solutions the alginate concentrations was adjusted so that the viscosity was around 300 mPas which gave spherically beads. The beads were made from solutions of 1.5% PRONOVA UP LVG alginate (FP-408-02, MW 219 kDa), 1.5% PRONOVA UP LVM alginate (FP-408-01, MW 222 kDa), 8.0% PRONOVA UP VLVG (FP-507-01, MW 41 kDa, purified from PROTANAL LFR 5/60) and 9.5% PRONOVA VLVG (FP-512-01, MW 25 kDa, manufactured by thermal depolymerization of dry powder; 80 degrees Celsius; treatment time 600 hours). Curve peaks (as indicated) are the result of the compression causing the beads to burst. Note that because of high viscosity it is, to our knowledge, not possible to make small spherical alginate beads under physiological conditions at concentrations above about 3% alginate when using LVG and LVM alginates. The mechanical strength of individual beads was measured with a texture analyzer from Stable Microsystems using a flat probe. The rate of compression was 0.1 mm/s and the compression force was measured as a function of compression. The data (Figure 4) shows that the final bursting force is much higher for the PRONOVA LVG and LVM alginate beads. However, the resistance towards compression is higher for both VLVG alginates below the bursting limit (peaks at about 1.4 mm compression). For lower degree of compression the data thus demonstrate that by using low MW alginates it may be possible to make alginate structures with increased rigidity without using solutions with very high viscosity.

### Example 4: Demonstration of in vivo degradability for an aleginate structure with defined low MW alginate fraction as an implantation material.

In this examples an alginate "self-gelling" composition was used which consists of a mixture of insoluble calcium alginate and a solution of sodium alginate. The formulation was chosen as it may easily be premixed and then injected with a syringe into different tissues before the solution forms an alginate hydrogel. In the testing a single subcutaneous or intramuscular administration was given to Sprague-Dawley rats.

For the study self-gel kits was first made, consisting of two 1 ml syringes with the alginate components which are connected with a 3 way-connector. The two syringes in each kit were filled with 0.2 ml 5% Ca-alginate (PRONOVA Ca M 45-75um, Batch: FU-606-01) and 0.8 ml 1.25% Na-alginate (PRONOVA VLVM, Batch: FP-604-02, manufactured by depolymerization from a commercial M-rich alginate using acid hydrolysis in solution at elevated temperature, pH 4.3, 75 degrees Celsius, 24 hours) respectively (in 4.6% D-mannitol). The Ca-alginate dispersion was sterilized by autoclaving (120°C at 20 minutes). The Na-alginate solution was sterilized by sterile filtration. The syringe filling was performed in a Safety Cabinet Class II and the kits were transferred to the animal facility and stored in a refrigerator until use. The MW of the PRONOVA VLVM alginate batch tested was measured to be around 35 kD (apparent viscosity 5.8 mPas in 1% solution at 20 degrees C measured using Brookfield viscosimetry).

Immediately before injection into the animals the two components were admixed by connecting the syringes and transferring the contents of one syringe into the other, then reversing the process for 10 number of mixes (5 each hand) starting with the syringe containing the largest volume (sodium alginate solution). The viscous solution was then injected into the animals immediately by using a 28G needle (0.50 or 0.25 ml injections). In the subcutaneous testing twelve males received an injection of 0.5 ml alginate self-gelling composition into the upper right dorsal scapula and, for comparison purposes, a single subcutaneous injection of the vehicle, 0.5 ml 4.6% mannitol solution, into the upper left dorsal scapula. Three animals were killed on Days 3, 15, 31 and 60 and the injection sites collected for histology. Similarly, another 9 males received a 0.25 ml intramuscular injection of the alginate self-gelling composition into the right hind limb, and 4.6% mannitol solution into the left hind limb. Three animals were killed on Days 3, 15 and 60 and the injection sites collected for histology. In both cases histological samples were stained with Alcian blue for visualization of alginate present in the tissue.

In both cases there were no adverse systemic signs noted during the observation period and bodyweight change was considered acceptable for rats of this age and strain. At the subcutaneous site receiving the alginate self-gelling composition, a small soft swelling was noted. Also at Day 3 and Day 15 the subcutaneous injection sites which received the alginate self-gelling composition showed subcutaneous gelatinous thickening. In one animal this was also noted for 19 days after injection. Although histological staining with Alcian blue verified the presence of moderate diffuse foreign alginate material at all 3 sites at 31 and 60 days after the injections, there were no macroscopic findings at necropsy on Day 29 and Day 60. Therefore the majority of the formulation had disappeared from the injection sites at this time. At the intramuscular injection sites there were no macroscopic findings at necropsy on day 3 demonstrating that the majority of the formulation had disappeared from the injection sites before the observation. Histological staining with Alcian blue, however, verified the presence of minimal diffuse or localized fascial foreign material at 2 out of 3 sites at day 3. At 15 days Alcian blue staining also verified the presence of minimal diffuse or moderate localized fascial foreign material at 2 out of 3 sites. In contrast there were no histological findings indicating the presence of alginate in the tissue at Day 60 indicating the total absence of alginate from the tissue.

In conclusion our observations demonstrate that the administered alginate gel, using an injectable alginate self-gel formulation with an alginate MW at about 35 kD, was able to degrade and disappear from the injection sites. Furthermore, the degradation rate was clearly tissue specific as the materials was observed to disappear faster at the intramuscular as compared to the subcutaneous site.

### THE FOLLOWING STATEMENTS DESCRIBE SOME EMBODIMENTS OF THE INVENTION

According to one embodiment of the invention there is provided a stable hydrogel comprising an alginate having a molecular weight less than 50,000 Daltons, wherein said alginate is present in an amount of at least 4% by weight of the gel.

The hydrogel may e.g., further comprise at least one of cells, pharmaceutically active agents, nutritional active agents, tissue, drugs, food, cosmetic agents, or radioactive isotope.

According to a second embodiment of the invention there is provided a hydrogel comprising a non-oxidized alginate having a molecular weight less than 50,000 Daltons and at least one of cells, pharmaceutically active agents, nutritional active agents, tissue, drugs, food, cosmetic agents, or radioactive isotopes, wherein said alginate is present in an amount of at least 4% by weight of the gel.

In the hydrogels of the invention said cells may e.g., comprise islet cells, kerotinocytes, hepatocytes, nephrons, chondrocytes, myoblasts, fibroblasts, neurons and said pharmaceutically active agents comprise antibiotics, cancer chemotherapeutics, morphine, growth factors and anti-infective agents.

The hydrogels of the invention may e.g., further comprise cells.

According to a third embodiment of the invention there is provided a hydrogel comprising a hydrogel comprising an alginate having a molecular weight greater than 10,000 Daltons and less than 75,000 Daltons, wherein the alginate is present in an amount of at least 4% by weight of the hydrogel, wherein said hydrogel further comprises cells or tissue.

Said alginate may e.g. have a molecular weight less than 50,000 Daltons, wherein the alginate is present in an amount of at least 4% by weight of the hydrogel, wherein said hydrogel further comprises cells or tissue.

In the hydrogels of the invention said alginate may e.g. be present in an amount of at least 5.0% by weight of the gel.

In the hydrogels of the invention said alginate may e.g. be present in an amount of at least 6.0% by weight of the gel.

In the hydrogels of the invention said alginate may e.g. be present in an amount of at least 7.0% by weight of the gel.

In the hydrogels of the invention said alginate may e.g. be present in an amount of at least 8.0% by weight of the gel.

The hydrogels of the invention may e.g. have a molecular weight less than 40,000 Daltons.

The hydrogels of the invention may e.g. have a molecular weight less than 30,000 Daltons.

The hydrogels of the invention may e.g. have a molecular weight less than 20,000 Daltons.

The hydrogels of the invention may e.g. further comprise at least one of calcium ions, strontium ions or barium ions ionically bound to said alginate.

In the hydrogels of the invention said alginate may e.g. comprise at least one of: (i) an alginate consisting of G-blocks, (ii) an alginate consisting of only M-blocks or (iii) an alginate consisting of MG-blocks.

In the hydrogels of the invention said alginate may e.g. comprise at least one alginate consisting of G-blocks.

In the hydrogels of the invention said alginate may e.g. comprise guluronic acid in an amount greater than 50% by weight of said alginate.

In the hydrogels of the invention said alginate may e.g. comprise guluronic acid in an amount less than 50% by weight of said alginate.

In the hydrogels of the invention said alginate may e.g. comprise at least one cell adhesion peptide covalently linked thereto.

In the hydrogels of the invention said cell adhesion peptide may e.g. be a peptide comprising RGD.

In the hydrogels of the invention said alginate may e.g. have an endotoxin level equal to or less than 1,000 EU/g.

The hydrogels of the invention may e.g. further comprise an alginate with a molecular weight of equal to or greater than 75,000 Daltons, wherein said alginate is present in an amount equal to or less than 2% of the gel.

The hydrogels of the invention may e.g. further comprise an alginate with a molecular weight of equal to or greater than 75,000 Daltons, wherein said alginate is present in an amount equal to or less than 1% of the gel.

According to a fourth embodiment of the invention there is provided a hydrogel formed by a method comprising:
a) forming a dispersion by mixing i) a solution comprising a soluble alginate with an insoluble gel particles or ii) immediately soluble alginate, insoluble gel particles and a solvent, and
b) dispensing the dispersion whereby the dispersion forms a hydrogel; wherein said soluble alginate has a molecular weight less than 75,000 Daltons, wherein the soluble alginate or immediately soluble alginate is present in an amount of at least 2.5% by weight of the gel.

Said soluble alginate or said immediately soluble alginate may e.g. be present in an amount of at least 4.0% by weight of the gel.

Said soluble alginate or said immediately soluble alginate may e.g. be present in an amount of at least 5.0% by weight of the gel.

Said soluble alginate or said immediately soluble alginate may e.g. be present in an amount of at least 6.0% by weight of the gel.

Said soluble alginate or said immediately soluble alginate may e.g. have a molecular weight less than 30,000 Daltons.

Said soluble alginate or said immediately soluble alginate may e.g. have a molecular weight less than 20,000 Daltons.

The hydrogels of the invention may e.g. further comprise at least one of calcium ions, strontium ions or barium ions ionically bound to said alginate.

Said soluble alginate or said immediately soluble alginate may e.g. comprise at least one of: (i) an alginate consisting of G-blocks, (ii) an alginate consisting of only M-blocks or (iii) an alginate consisting of MG-blocks.

Said soluble alginate or said immediately soluble alginate may e.g. comprise at least one alginate consisting of G-blocks.

Said soluble alginate or said immediately soluble alginate may e.g. comprise guluronic acid in an amount greater than 50% by weight of said alginate.

Said soluble alginate or said immediately soluble alginate may e.g. comprise at least one cell adhesion peptide covalently linked thereto.

Said soluble alginate or said immediately soluble alginate, and said insoluble gel particles may e.g. each have an endotoxin level equal to or less than 1,000 EU/g.

The hydrogels of the invention may e.g. further comprise at least one of cells, pharmaceutically active agents, nutritional active agents, tissue, drugs, food, cosmetic agents, or radioactive isotope.

Said cells may e.g. comprise islet cells, kerotinocytes, hepatocytes, nephrons, chondrocytes, myoblasts, fibroblasts, neurons and said pharmaceutically active agents comprise antibiotics, cancer chemotherapeutics, morphine, growth factors and anti-infective agents.

The hydrogels of the invention may e.g. further comprise cells.

According to a fifth embodiment of the invention there is provided a bead comprising a hydrogel of the invention.

The bead may e.g. comprise an alginate having a molecular weight less than 75,000 Daltons, wherein said alginate is present in an amount of at least 4% by weight of the gel.

Said alginate may e.g. be present in an amount of at least 5.0% by weight of the gel.

Said alginate may e.g. be present in an amount of at least 6.0% by weight of the gel.

Said alginate may e.g. be present in an amount of at least 7.0% by weight of the gel.

The bead of the invention may e.g. have a molecular weight less than 30,000 Daltons.

The bead of the invention may e.g. have a molecular weight less than 20,000 Daltons.

The bead of the invention may e.g. further comprise at least one of calcium ions, strontium ions or barium ions ionically bound to said alginate.

Said alginate may e.g. comprise at least one of: (i) an alginate consisting of G-blocks, (ii) an alginate consisting of only M-blocks or (iii) an alginate consisting of MG-blocks.

Said alginate may e.g. comprise at least one alginate consisting of G-blocks.

Said alginate may e.g. comprise guluronic acid in an amount greater than 50% by weight of said alginate.

Said alginate may e.g. comprise at least one cell adhesion peptide covalently linked thereto.

Said cell adhesion peptide may e.g. be a peptide comprising RGD.

Said alginate may e.g. have an endotoxin level equal to or less than 1,000 EU/g.

A bead of the invention, may e.g. further comprise at least one of cells, pharmaceutically active agents, nutritional active agents, tissue, drugs, food, cosmetic agents, or radioactive isotope.

Said cells may e.g. comprise islet cells, kerotinocytes, hepatocytes, nephrons, chondrocytes, myoblasts, fibroblasts, neurons and said pharmaceutically active agents comprise antibiotics, cancer chemotherapeutics, morphine, growth factors and anti-infective agents.

The bead of the invention may e.g., further comprise cells.

According to a sixth embodiment of the invention there is provided a method of making a hydrogel or bead, comprising the steps of preparing a solution of the alginate of the invention, and adding said alginate solution to a solution comprising gelling cations to form said hydrogel.

According to a seventh embodiment of the invention there is provided a method of making a hydrogel, comprising mixing a compound containing gelling cations into a solution containing the alginate of the invention, where said cations are released at a desired rate in said alginate solution to form said hydrogel.

Said compound may e.g. be at least one of calcium carbonate, calcium containing liposome, calcium sulfate, calcium phosphate, calcium lactate or calcium citrate.

Said gelling cations may e.g. comprise calcium ions, barium ions, strontium ions, or combination thereof.

According to an eighth embodiment of the invention there is provided the product of the method of the sixth or seventh embodiment of the invention.

According to a ninth embodiment of the invention there is provided an implantable medical device comprising a hydrogel of the invention.

According to a tenth embodiment of the invention there is provided a method of implanting a bead or implantable medical device in patient in need thereof, comprising implanting a bead of the invention or an implantable medical device of the invention.

According to an eleventh embodiment of the invention there is provided a method of implanting cells in need thereof, comprising implanting a hydrogel of the invention or a bead of the invention, or the implantable medical device of the invention, wherein said hydrogel of said hydrogel, bead, or implantable medical device further comprises cells.

According to a twelfth embodiment of the invention there is provided a drug delivery formulation comprising a hydrogel of the invention or a bead of the invention, or the implantable medical device of the invention.

According to a thirteenth embodiment of the invention there is provided a contrast agent or radio-opaque material comprising an alginate having a molecular weight less than 75.000 Daltons and barium ionically bound to said alginate, wherein: said alginate is present in an amount of at least 2.5% by weight of the gel; and said barium in the hydrogel is used as a radio-opaque material.

In said contrast agent or radio-opaque material of the invention, said alginate may e.g. have a molecular weight less than 50,000.

In said contrast agent or radio-opaque material, of the invention said alginate may e.g. have a molecular weight less than 30,000.

In said contrast agent or radio-opaque material, of the invention said alginate may e.g. be present in an amount of at least 5% by weight of the gel.

In said contrast agent or radio-opaque material, of the invention said alginate may e.g. be present in an amount of at least 7% by weight of the gel.

According to a fourteenth embodiment of the invention there is provided a method of blocking blood vessels by using a hydrogel of the invention, or beads of the invention, wherein said hydrogel or said beads are bioresorbable.

According to a fifteenth embodiment of the invention there is provided an embolic therapeutic composition comprising a hydrogel of the invention, or a bead of the invention; and wherein said hydrogel or said beads are bioresorbable.

According to a sixteenth embodiment of the invention there is provided a method of forming a hydrogel of the invention, comprising:
a) forming a dispersion by mixing i) a solution comprising a soluble alginate with an insoluble gel particles or ii) immediately soluble alginate, insoluble gel particles and a solvent, and
b) dispensing the dispersion whereby the dispersion forms a hydrogel; wherein said soluble alginate has a molecular weight less than 75,000 Daltons, wherein the soluble alginate or immediately soluble alginate is present in an amount of at least 2.5% by weight of the hydrogel.

## Claims

1. A stable hydrogel comprising a non-oxidised alginate having a molecular weight less than 50.000 Daltons and at least one of calcium ions, strontium ions or barium ions ionically bound to said alginate, wherein said alginate is present in an amount of at least 4% by weight of the gel.

2. A hydrogel according to claim 1, further comprising at least one of cells (with the exception of embryonic stem cells, pharmaceutically active agents, nutritional active agents, tissue, drugs, food, cosmetic agents, or radioactive isotope.

3. The hydrogel according to claim 1 or 2, wherein said cells comprise islet cells, kerotinocytes, hepatocytes, nephrons, chondrocytes, myoblasts, fibroblasts, neurons and said pharmaceutically active agents comprise antibiotics, cancer chemotherapeutics, morphine, growth factors and anti-infective agents.

4. The hydrogel according to any one of claims 1 to 3, further comprising cells.

5. The hydrogel according to any one of claims 1 to 4, wherein said alginate is present in an amount of at least 5.0% by weight of the gel.

6. The hydrogel according to any one of claims 1 to 4, wherein said alginate, is present in an amount of at least 6.0% by weight of the gel.

7. A hydrogel according two any one of claims 1 to 4, wherein said alginate is present in an amount of at least 70% by weight of the gel.

8. A hydrogel according to any one of claims I to 4, wherein said alginate is present in an amount of at 8.0% by of the gel.

9. The hydrogel according two any of claims I two 4, having a molecular weight less than 40,000 Daltons,

10. The hydrogel according to any one of claims 1 to 4, having a molecular weight less than 30,000 Daltons.

11. The hydrogel According to any one of claims 1 to 4, having a molecular weight less than 20,000 Daltons.

12. The hydrogel according to any one off claims 1 to 11 wherein said alginate comprises at least one of: (i) an alginate consisting of G-blocks, (ii) an alginate consisting of only M-blocks or (iii) an alginate consisting of MG-blocks.

13. The hydrogel According two any one of claims 1 to 11 wherein said alginate comprises at least one alginate consisting of G-blocks.

14. The hydrogel according to claims 1 to 11, wherein said alginate comprises guluronic acid in an amount greater than 50% by weight of said alginate.

15. The hydrogel according to claims 1 to 11, wherein said alginate comprise guluronic acid in an amount less than 50% by weight of said alginate.

16. The hydrogel according to 1 to 11, wherein said alginate comprises at least once cell adhesion peptide covalently linked thereto.

17. The hydrogel according to 1 to 11, wherein said cell adhesion peptide is a peptide comprising RGD.

18. The hydrogel according to claims 1 to 17, wherein said alginate has an endotoxin level equal to or less than 1 ,000 EU/g.

19. A hydrogel according to any one of claims 1 to 18, further comprising an alginate with a molecular weight of equal to or greater than 75,000 Daltons, wherein said alginate is present in an amount equal to or less than 2% of the gel.

20. A hydrogel according to any one of claims 1 to 18, further comprising an alginate with a molecular weight of equal to or greater than 75,000 Daltons, wherein said alginate is present in an amount equal to or less than 1% of the gel.

21. A hydrogel formed by a method comprising:
a) forming a dispersion by mixing i) a solution comprising a soluble alginate with an insoluble gel particles or ii) immediately soluble alginate, insoluble gel particles and a solvent, and
b) dispensing the dispersion thereby the dispersion forms a hydrogel; wherein soluble alginate has a molecular weight less than 75,000 Daltons, wherein the soluble alginate or immediately soluble alginate is present in an amount of at least 2.5% by weight of the gel.

22. A bead comprising a hydrogel according to any one of claims 1 to 20.

23. A method of making a hydrogel or bead, comprising the steps of preparing a solution of the alginate in any one of claims 1 to 20, and adding said alginate solution to a solution comprising gelling cations to form said hydrogel.

24. A method of making hydrogel, comprising mixing a compound containing gelling cations into a solution containing the alginate of claims 1 to 20, where said cations are released at a desired rate in said hydrogel.

25. A implantable medical device comprising a hydrogel according to any one of claims 1 to 20.

26. A method of implanting a bead or implantable medical device in patient in need thereof, comprising implanting a bead according to claims 22 or an implantable medical device according to claims 25.

27. A method of implanting cells in need thereof, comprising: implanting a hydrogel according to any of claims 1 to 20 or a bead acoording to claim 22 or the implantable medical device according to claim 25, wherein said hydrogel of said hydrogel, bead, or iinplantable medical device further comprises cells.

28. A drug delivery formulation comprising a hydrogel according to any one of claims 1 to 20 or a bead according to claim 22, or the implantable medical device according to claim 25.

29. A contrast agent or radio-opaque material comprising an alginate having a molecular weight less than 75,000 Daltons and barium ironically bound to said alginate; wherein: said alginate is present in an amount of at least 2.5% by weight of the gel; and said barium in the hydrogel is used as a radio-opaque material.

30. A hydrogel according to any one of claims 1 to 20, or beads according to claim 22, for use in blocking blood vessels wherein said hydrogel or said beads are bioresorbable.

31. An embolic therapeutic composition comprising a hydrogel according to any one of claims I to 20, or a bead according to claim 22, and wherein said hydrogel or said beads are bioresorbable.

32. A method of forming a hydrogel according to any one of claims 19 to 21. comprising:
a) forming a dispersion by mixing
i) a solution comprising a soluble alginate with an insoluble gel particles or
ii) immediately soluble alginate, insoluble gel particles and a solvent,
and
b) dispensing the dispersion thereby the dispersion forms a hydrogel;
wherein said soluble alginate has a molecular weight less than 75,000 Daltons, wherein the soluble, alginate or immediately soluble alginate is present in an amount of at least 2.5% by weight of the hydrogel.

## Patentansprüche

1. Stabiles Hydrogel, das Folgendes umlasst: ein nicht oxidiertes Alginat mit einem Molekulargewicht von kleiner als 50 000 Dalton und mindestens eines von Calciumionen, Strontiumionen oder Bariumionen, die ionisch zum genannten Alginat gebunden sind, worin das genannte Alginat in einer Menge von mindestens 4 Gewichts-% des Gels vorliegt.

2. Hydrogel nach Anspruch 1, das weiter mindestens eines von Folgenden umfasst: Zellen (mit Ausnahme von embryonalen Stammzellen), pharmazeutische Wirkstoffe, Nahrungswirkstoffe, Gewebe, Arzneimittel, Nahrungsmittel, kosmetische Mittel oder radioaktives Isotop.

3. Hydrogel nach Anspruch 1 oder 2, worin die genannten Zellen Folgende umfassen: Inselzellen, Keratinozyten, Hepatozyten, Nephrone, Chondrozyten, Myoblasten, Fibroblasten, Neurone, und die genannten pharmazeutischen Wirkstoffe Folgende umfassen: Antibiotika, Krebs-Chemotherapeutika, Morphin, Wachstumsfaktoren und Antiinfektiva.

4. Hydrogel nach einem der Ansprüche 1 bis 3, das weiter Zellen umfasst.

5. Hydrogel nach einem der Ansprüche 1 bis 4, worin das genannte Alginat in einer Menge von mindestens 5,0 Gewichts-% des Gels vorliegt.

6. Hydrogel nach einem der Ansprüche 1 bis 4, worin das genannte Alginat in einer Menge von mindestens 6,0 Gewichts-% des Gels vorliegt.

7. Hydrogel nach einem der Ansprüche 1 bis 4, worin das genannte Alginat in einer Menge von mindestens 7,0 Gewichts-% des Gels vorliegt.

8. Hydrogel nach einem der Ansprüche 1 bis 4, worin das genannte Alginat in einer Menge von mindestens 8,0 Gewichts-% des Gels vorliegt.

9. Hydrogel nach einem der Ansprüche 1 bis 4, das ein Molekulargewicht von kleiner als 40 000 Dalton aufweist.

10. Hydrogel nach einem der Ansprüche 1 bis 4, das ein Molekulargewicht von kleiner als 30 000 Dalton aufweist.

11. Hydrogel nach einem der Ansprüche 1 bis 4, das ein Molekulargewicht von kleiner als 20 000 Dalton aufweist.

12. Hydrogel nach einem der Ansprüche 1 bis 11, worin das genannte Alginat mindestens eines von Folgenden umfasst: (i) ein Alginat, das aus G-Blöcken besteht. (ii) ein Alginat, das nur aus M-Blöcken besteht, oder (iii) ein Alginat, das aus MG-Blöcken besteht.

13. Hydrogel nach einem der Ansprüche 1 bis 11, worin das genannte Alginat mindestens ein Alginat umfasst, das aus G-Blöcken besteht.

14. Hydrogel nach den Ansprüchen 1 bis 11, worin das genannte Alginat Guluronsäure in einer Menge von mehr als 50 Gewichts-% des genannten Alginats umfasst.

15. Hydrogel nach den Ansprüchen 1 bis 11, worin das genannte Alginat Guluronsäure in einer Menge von weniger als 50 Gewichts-% des genannten Alginats umfasst.

16. Hydrogel nach den Ansprüchen 1 bis 11, worin das genannte Alginat mindestens ein Zelladhäsionspeptid umfasst, das damit kovalent verknüpft ist.

17. Hydrogel nach den Ansprüchen 1 bis 11, worin das genannte Zelladhäsionspeptid ein Peptid ist, das RGD umfasst.

18. Hydrogel nach den Ansprüchen 1 bis 17, worin das genannte Alginat ein Endotoxin-Niveau hat, das gleich oder kleiner als 1 000 EU/g ist.

19. Hydrogel nach einem der Ansprüche 1 bis 18, das weiter ein Alginat mit einem Molekulargewicht von gleich oder größer als 75 000 Dalton umfasst, worin das genannte Alginat in einer Menge von gleich oder weniger als 2 % des Gels vorliegt.

20. Hydrogel nach einem der Ansprüche 1 bis 18, das weiter ein Alginat mit einem Molekulargewicht von gleich oder größer als 75 000 Dalton umfasst, worin das genannte Alginat in einer Menge von gleich oder weniger als 1 % des Gels vorliegt.

21. Hydrogel, das durch ein Verfahren gebildet wurde, das Folgendes umfasst:
a) Bilden einer Dispersion durch Mischen von i) einer Lösung, die ein lösliches Alginat umfasst, mit unlöslichen Gelpartikeln oder ii) sofort löslichem Alginat, unlöslichen Gelpartikeln und einem Lösungsmittel, und
b) Abgeben der Dispersion, wodurch die Dispersion ein Hydrogel bildet; worin das genannte lösliche Alginat ein Molekulargewicht von kleiner als 75 000 Dalton aufweist, worin das lösliche Alginat oder sofort lösliche Alginat in einer Menge von mindestens 2,5 Gewichts-% des Gels vorliegt.

22. Kügelchen, das ein Hydrogel nach einem der Ansprüche 1 bis 20 umfasst.

23. Verfahren zur Herstellung eines Hydrogels oder Kügelchens, das die folgenden Schritte umfasst: Herstellen einer Lösung des Alginats nach einem der Ansprüche 1 bis 20 und Zugeben der genannten Alginatlösung zu einer Lösung, die Kationen zur Gelbildung umfasst, um das genannte Hydrogel zu bilden.

24. Verfahren zur Herstellung eines Hydrogels, das das Mischen einer Verbindung, die Kationen zur Gelbildung enthält, in einer Lösung, die das Alginat nach den Ansprüchen 1 bis 20 enthält, umfasst, wo die genannten Kationen in einer gewünschten Geschwindigkeit im genannten Hydrogel freigesetzt werden.

25. Implantierbares Medizinprodukt, das ein Hydrogel nach einem der Ansprüche 1 bis 20 umfasst.

26. Verfahren zum Implantieren eines Kügelchens oder implantierbaren Medizinprodukts in einem Patienten, der dieses benötigt, das das Implantieren eines Kügelchens nach Anspruch 22 oder eines implantierbaren Medizinprodukts nach Anspruch 25 umfasst.

27. Verfahren zum Implantieren von Zellen, die dies benötigen, das das Implantieren eines Hydrogels nach einem der Ansprüche 1 bis 20 oder eines Kügelchens nach Anspruch 22 oder des implantierbaren Medizinprodukts nach Anspruch 25 umfasst, worin das genannte Hydrogel von dem genannten Hydrogel, Kügelchen oder implantierbaren Medizinprodukt weiter Zellen umfasst.

28. Arzneimittelabgabeformulierung, die ein Hydrogel nach einem der Ansprüche 1 bis 20 oder ein Kügelchen nach Anspruch 22 oder das implantierbare Medizinprodukt nach Anspruch 25 umfasst.

29. Kontrastmittel oder radioopaker Stoff, das ein Alginat mit einem Molekulargewicht von kleiner als 75 000 Dalton und Barium, das ionisch an das genannte Alginat gebunden ist, umfasst, worin das genannte Alginat in einer Menge von mindestens 2,5 Gewichts-% des Gels vorliegt und das genannte Barium im Hydrogel als radioopaker Stoff verwendet wird.

30. Hydrogel nach einem der Ansprüche 1 bis 20 oder Kügelchen nach Anspruch 22 zur Verwendung bei der Blockierung von Blutgetäßen, worin das genannte Hydrogel oder die genannten Kügelchen bioresorbierbar sind.

31. Embolische therapeutische Zusammensetzung, die ein Hydrogel nach einem der Ansprüche 1 bis 20 oder ein Kügelchen nach Anspruch 22 umfasst und worin das genannte Hydrogel oder die genannten Kügelchen bioresorbierbar sind.

32. Verfahren zur Bildung eines Hydrogels nach einem der Ansprüche 19 bis 21, das Folgendes umfasst:
a) Bilden einer Dispersion durch Mischen von
i) einer Lösung, die ein lösliches Alginat umfasst, mit unlöslichen Gelpartikeln oder
ii) sofort löslichem Alginat, unlöslichen Gelpartikeln und einem Lösungsmittel, und
b) Abgeben der Dispersion, wodurch die Dispersion ein Hydrogel bildet;
worin das genannte lösliche Alginat ein Molekulargewicht von kleiner als 75 000 Dalton aufweist, worin das lösliche Alginat oder sofort lösliche Alginat in einer Menge von mindestens 2,5 Gewichts-% des Hydrogels vorliegt.

## Revendications

1. Hydrogel stable comprenant un alginate non oxydé ayant un poids moléculaire inférieur à 50 000 Daltons et au moins un parmi des ions calcium, des ions strontium ou des ions baryum liés de manière ionique audit alginate, où ledit alginate est présent selon une quantité d'au moins 4% en poids du gel.

2. Hydrogel selon la revendication 1, comprenant en outre au moins un ou une parmi des cellules (à l'exception de cellules souches embryonnaires), des agents pharmaceutiquement actifs, des agents actifs nutritionnels, des tissus, des médicaments, des aliments, des agents cosmétiques ou des isotopes radioactifs.

3. Hydrogel selon la revendication 1 ou 2, dans lequel lesdites cellules comprennent des îlots de Langerhans, des kératinocytes, des hépatocytes, des néphrons, des chondrocytes, des myoblastes, des tibroblastes, des neurones, et lesdits agents pharmaceutiquement actifs comprennent des antibiotiques, des agents chimiothérapeutiques anticancéreux, de la morphine, des facteurs de croissance et des agents anti-infectieux.

4. Hydrogel selon l'une quelconque des revendications 1 à 3, comprenant en outre des cellules.

5. Hydrogel selon l'une quelconque des revendications 1 à 4, dans lequel ledit alginate est présent selon une quantité d'au moins 5,0% en poids du gel.

6. Hydrogel selon l'une quelconque des revendications 1 à 4, dans lequel ledit alginate est présent selon une quantité d'au moins 6,0% en poids du gel.

7. Hydrogel selon l'une quelconque des revendications 1 à 4, dans lequel ledit alginate est présent selon une quantité d'au moins 7,0% en poids du gel.

8. Hydrogel selon l'une quelconque des revendications 1 à 4, dans lequel ledit alginate est présent selon une quantité d'au moins 8,0% en poids du gel.

9. Hydrogel selon l'une quelconque des revendications 1 à 4, ayant un poids moléculaire inférieur à 40 000 Daltons.

10. Hydrogel selon l'une quelconque des revendications 1 à 4, ayant un poids moléculaire inférieur à 30 000 Daltons.

11. Hydrogel selon l'une quelconque des revendications 1 à 4, ayant un poids moléculaire inférieur à 20 000 Daltons.

12. Hydrogel selon l'une quelconque des revendications 1 à 11, dans lequel ledit alginate comprend au moins l'un parmi : (i) un alginate constitué de blocs G ; (ii) un alginate constitué seulement de blocs M ; ou (iii) un alginate constitué de blocs MG.

13. Hydrogel selon l'une quelconque des revendications 1 à 11, dans lequel ledit alginate comprend au moins un alginate constitué de blocs G.

14. Hydrogel selon les revendications 1 à 11, dans lequel ledit alginate comprend de l'acide guluronique selon une quantité supérieure à 50% en poids dudit alginate.

15. Hydrogel selon les revendications 1 à 11, dans lequel ledit alginate comprend de l'acide guluronique selon une quantité inférieure à 50% en poids dudit alginate.

16. Hydrogel selon les revendications 1 à 11, dans lequel ledit alginate comprend au moins un peptide d'adhésion cellulaire y étant lié de manière covalente.

17. Hydrogel selon les revendications 1 à 11, dans lequel ledit peptide d'adhésion cellulaire est un peptide comprenant RGD.

18. Hydrogel selon les revendications 1 à 17, dans lequel ledit alginate possède un taux d'endotoxine inférieur ou égal à 1000 UE/g.

19. Hydrogel selon l'une quelconque des revendications 1 à 18, comprenant en outre un alginate ayant un poids moléculaire supérieur ou égal à 75 000 Daltons, où ledit alginate est présent selon une quantité inférieure ou égale à 2% du gel.

20. Hydrogel selon l'une quelconque des revendications 1 à 18, comprenant en outre un alginate ayant un poids moléculaire supérieur ou égal à 75 000 Daltons, où ledit alginate est présent selon une quantité inférieure ou égale à 1% du gel.

21. Hydrogel formé par une méthode comprenant :
a) la formation d'une dispersion par le mélange i) d'une solution comprenant un alginate soluble avec des particules de gel insolubles ou ii) d'alginate immédiatement soluble, de particules de gel insolubles et d'un solvant, et
b) la distribution de la dispersion, ce par quoi la dispersion forme un hydrogel ; où ledit alginate soluble possède un poids moléculaire inférieur à 75 000 Daltons, où l'alginate soluble ou l'alginate immédiatement soluble est présent selon une quantité d'au moins 2.5% en poids du gel.

22. Bille comprenant un hydrogel selon l'une quelconque des revendications 1 à 20.

23. Méthode de préparation d'un hydrogel ou d'une bille, comprenant les étapes consistant à préparer une solution de l'alginate selon l'une quelconque des revendications 1 à 20, et ajouter ladite solution d'alginate à une solution comprenant des cations de gélification afin de former ledit hydrogel.

24. Méthode de préparation d'un hydrogel, comprenant le mélange d'un composé contenant des cations de gélification dans une solution contenant l'alginate selon les revendications 1 à 20, où lesdits cations sont libérés à la vitesse souhaitée dans ledit hydrogel.

25. Dispositif médical implantable comprenant un hydrogel selon l'une quelconque des revendications 1 à 20.

26. Méthode d'implantation d'une bille ou d'un dispositif médical implantable chez un patient en ayant besoin, comprenant l'implantation d'une bille selon la revendication 22 ou d'un dispositif médical implantable selon la revendication 25.

27. Méthode d'implantation de cellules en ayant besoin, comprenant l'implantation d'un hydrogel selon l'une quelconque des revendications 1 à 20 ou d'une bille selon la revendication 22, ou du dispositif médical implantable selon la revendication 25, dans laquelle ledit hydrogel dudit hydrogel, de ladite bille ou dudit dispositif médical implantable comprend en outre des cellules.

28. Formulation d'administration de médicament, comprenant un hydrogel selon l'une quelconque des revendications 1 à 20, ou une bille selon la revendication 22, ou le dispositif médical implantable selon la revendication 25.

29. Agent de contraste ou matériau radio-opaque comprenant un alginate ayant un poids moléculaire inférieur à 75 000 Daltons et du baryum lié de manière ionique audit alginate, où ledit alginate est présent selon une quantité d'au moins 2,5% en poids du gel ; et ledit baryum dans l'hydrogel est utilisé comme matériau radio-opaque.

30. Hydrogel selon l'une quelconque des revendications 1 à 20, ou billes selon la revendication 22, pour une utilisation dans le blocage de vaisseaux sanguins, où ledit hydrogel ou lesdites billes sont biorésorbables.

31. Composition thérapeutique embolique comprenant un hydrogel selon l'une quelconque des revendications 1 à 20, ou une bille selon la revendication 22, et où ledit hydrogel ou lesdites billes sont biorésorbables.

32. Méthode de formation d'un hydrogel selon l'une quelconque des revendications 19 à 21, comprenant :
a) la formation d'une dispersion, par le mélange
i) d'une solution comprenant un alginate soluble avec des particules de gel insolubles, ou
ii) d'alginate immédiatement soluble, de particules de gel insolubles et d'un solvant, et
b) la distribution de la dispersion, ce par quoi la dispersion forme un hydrogel ; où ledit alginate soluble possède un poids moléculaire inférieur à 75 000 Daltons, où l'alginate soluble ou l'alginate immédiatement soluble est présent selon une quantité d'au moins 2,5% en poids de l'hydrogel.
